# EUROPEAN PATENT APPLICATION

(11) **EP 2 746 407 A1**
(43) Date of publication of application: **25.06.2014**
(21) Application number: 12197790.4
(22) Date of filing: 18.12.2012
(51) Int. Cl.: C12R 1/66

(54) **Novel Aspergillus sp. strain NBIMCC 8735 and method of producing chitinase utilizing the same**

(71) Applicant: EADS Deutschland GmbH, 85521 Ottobrunn (DE)
(72) Inventor: Raps, Dominik, 86633 Neuburg (DE); Krastanov, Albert, 4002 Plovdiv (BG); Nedelcheva, Plamena, 9300 Dobrich (BG); Draganova, Donka, 4002 Plovdiv (BG)
(74) Representative: Maiwald Patentanwalts GmbH

(57) **Abstract**

The present invention pertains to a novel *Aspergillus sp.* strain, wherein the strain is *Aspergillus flavus* H5, as deposited in the National Bank for Industrial Microorganisms and Cell Cultures (NBIMCC) - Bulgaria under the access no. NBIMCC 8735, and to a method for producing chitinase utilizing the same. Moreover, the present invention pertains to the chitinase produced by said strain and to the use of said chitinase in a temporary or permanent anti-contamination coating system that can be applied to a surface for the detachment of chitin-containing debris (e.g., insect debris) from said surface, e.g., from an airfoil.

## Description

### FIELD OF THE INVENTION

The present invention pertains to a novel *Aspergillus sp.* strain, wherein the strain is *Aspergillus flavus* H5, as deposited in the National Bank for Industrial Microorganisms and Cell Cultures (NBIMCC) - Bulgaria under the access no. NBIMCC 8735 by Biodinamika Ltd., GK Trakia No. 398, entr. 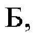 Plovdiv, 4000, Bulgaria, and to a method for producing chitinase utilizing the same. Moreover, the present invention pertains to the chitinase produced by said strain and to the use of said chitinase in a temporary or permanent anti-contamination coating system that can be applied to a surface for the detachment of chitin-containing debris (e.g., insect debris) from said surface, preferably from a flow surface (in particular, a laminar flow surface), more preferably from an airfoil, a hydrofoil, or a blade of a wind energy conversion system, even more preferably from an airfoil, and most preferably from a laminar airfoil. In particular, the present invention pertains to the use of said chitinase in a temporary cleaning composition for removing and/or preventing chitin-containing debris on said surfaces.

### BACKGROUND OF THE INVENTION

Chitin is a typical marine polysaccharide, the second most abundant polysaccharide in nature and an abundant biomass resource. Chitin is an insoluble linear β-1,4-*N-*acetylglucosamine polymer, and the main component of the cell walls of fungi, exoskeletons of arthropods, such as insects and crustaceans, including crabs, lobsters and shrimps, radulas of mollusks, beaks of cephalopods, including squid and octopuses, and some algae. It has been estimated that the worldwide annual recovery of chitin from processing marine invertebrates is 3.7x10⁴ metric tons. In terms of structure, chitin may be compared to the polysaccharide cellulose and, in terms of function, to the protein keratin.

Chitinases (1,4-beta-poly-N-acteylglucosaminidases, EC 3.2.1.14) are hydrolytic enzymes that break down chitin, thereby playing an important role in the decomposition of chitin, and potentially in the utilization of chitin as a renewable resource.

There is a growing interest in the derivatives obtained from chitin hydrolysis, which are chitooligomers, *N*-acetyl-D-glucosamine (GlcNAc) and glucosamine (GlcN). The monomers GlcNAc and GlcN have been applied in the treatment of osteoarthritis and inflammatory bowel disease, or as nutraceutical agents for patients with osteoarthritis and inflammatory bowel disease. In contrast to glucosamine hydrochloride or glucosamine sulfate, both of which have a bitter taste, *N*-acetyl-D-glucosamine has a sweet taste that facilitates its use in daily consumption.

Moreover, there is a wide and almost unexplored field in the use of chitooligosaccharides derived from enzymatic hydrolysis of chitin, which could be used for the production of high added value products. The study of chitooligosaccharides, in particular of their potential biological activities, constitutes an area of growing interest. For example, the oligo-*N*-acetyl-D-glucosamines (GlcNAc)₆ and (GlcNAc)₇ show antitumor and antimicrobial activities.

However, *N*-acetyl-D-glucosamine has not been widely commercialized mainly due to the lack of an economical process for its production. The acidic hydrolysis of chitin using concentrated hydrochloric acid is inefficient and possesses environmental and technical concerns. Enzymatic methods have been proposed as alternatives to the conventional chemical processes, because of their ability to produce *N*-acetyl-D-glucosamine under mild and environmentally friendly conditions.

Chitinases have received increasing attention because of their broad applications in the field of medicine, agriculture, biotechnology, waste management and industry. They have antifungal, hypocholesterolemic, and antihypertensive activity, and are used, for example, as biopesticides and food quality enhancers. Chitinases are also extensively used in biological research for the generation of fungal protoplasts due to their ability to degrade fungal cell walls. The hydrolytic property of chitinases makes them an attractive alternative as an environmentally safe biocontrol agent. The characteristics of high pH tolerance and salinity tolerance of marine microbial chitinase may contribute to its special biotechnological potentiality.

Many studies exist on the application of chitinase in the treatment of cancer. Commercial bacterial chitinases and a recombinant chitinase from plant have been found to be able to selectively lyse cancer cells, but not normal cells in culture. Lysis of solid tumors, as well as the cure of mice carrying cancer, after injecting commercial bacterial chitinases, have also been reported.

Chitinase producing organisms have also been used for inhibiting the growth of phytopathogens.

Furthermore, as there are enormous chitin accumulations causing remarkable environmental problems worldwide, chitinases may find important industrial applications for the degradation of chitin.

There is also a need to reduce fuel consumption in aviation. A reduction of turbulent flows on the aircraft surface results in less air resistance, and thus lower fuel consumption. Therefore, the outer shell of an aircraft, i.e. wing, fuselage and tail, are designed in such a way that preferably laminar flows occur. In practice, however, these laminar flows are disrupted by contaminants on the outer shell. By the deposition of contaminants on the outer shell, a rough surface is formed resulting in undesirable turbulences, and hence resulting in higher fuel consumption. Besides water, that deposits in the form of ice, predominantly insect deposition occurs, especially during the first phase of flight, i.e. between take off and reaching cruising altitude. Insects are mainly composed of two components, exoskeleton and hemolymph. The exoskeleton consists mainly of the polysaccharide chitin and the structural proteins sclerotin and resilin. If an insect hits a surface of an aircraft with high speed, the exoskeleton breaks and hemolymph is discharged, thereby depositing the insect on the surface due to the surface adhesion effect. Therefore, an enzyme, enzyme mixture, or composition comprising an enzyme, is needed that has a particularly high enzyme activity in order to decompose chitin, and hence remove the insect deposits from surfaces of aircrafts.

However, chitinases have not been engaged at a huge commercial scale yet, due to their high production cost. Currently commercially available chitinases are very expensive (e.g., 5740 € for 1 g chitinase from *Streptomyces griseus,* C6137, Sigma-Aldrich). In addition, the selection of commercially available chitinases is limited. Furthermore, many chitinases show a limited activity, as well as stability.

Therefore, there is still a great need for chitinases which can be easily produced at low cost and exhibit high activity and very good stability in various media and at various temperatures.

In view of the above, it is an object of the present invention to provide an appropriate microbial strain, which can produce a chitinase pursuant to the demand in the field of medicine, agriculture, biotechnology, waste management and industry, in particular in the field of aerodynamics.

In particular, it is an object of the present invention to provide a novel microbial strain and a method for producing chitinase utilizing the same.

Another object of the present invention is to provide a chitinase produced by the novel microbial strain.

Another object of the present invention is to provide a chitinase for use in a temporary or permanent anti-contamination coating system that can be applied to a surface for the detachment of chitin-containing debris (e.g., insect debris) from said surface, preferably from a flow surface (in particular, a laminar flow surface), more preferably from an airfoil, a hydrofoil, or a blade of a wind energy conversion system, even more preferably from an airfoil, and most preferably from a laminar airfoil. In particular, an object of the present invention is to provide a chitinase for use in a temporary cleaning composition for removing and/or preventing chitin-containing debris on said surfaces.

Another object of the present invention is to provide a coated surface, wherein the surface is coated with a composition comprising a chitinase, preferably a chitinase of the present invention. The coated surface is preferably a flow surface (in particular a laminar flow surface), more preferably an airfoil, a hydrofoil, or a blade of a wind energy conversion system, even more preferably an airfoil and most preferably a laminar airfoil.

These and other objectives as they will become apparent from the ensuing description of the invention are solved by the present invention as described in the independent claims. The dependent claims pertain to preferred embodiments.

### SUMMARY OF THE INVENTION

In one aspect, the present invention provides a novel *Aspergillus sp.* strain, wherein the strain is *Aspergillus flavus* H5, as deposited in the National Bank for Industrial Microorganisms and Cell Cultures (NBIMCC) - Bulgaria under the access no. NBIMCC 8735 by Biodinamika Ltd., GK Trakia No. 398, entr. 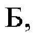 Plovdiv, 4000, Bulgaria, and a method for producing chitinase utilizing the same.

In another aspect, the present invention provides a chitinase, defined as chitinase H5, produced by said strain, and its use. In a specific aspect, said chitinase is used in a temporary or permanent anti-contamination coating system that can be applied to a surface for the detachment of chitin-containing debris (e.g., insect debris) from said surface, preferably from a flow surface (in particular, a laminar flow surface), more preferably from an airfoil, a hydrofoil, or a blade of a wind energy conversion system, even more preferably from an airfoil, and most preferably from a laminar airfoil. In particular, the present invention provides a chitinase for use in a temporary cleaning composition for removing and/or preventing chitin-containing debris on said surfaces.

### BRIEF DESCRIPTION OF THE FIGURES

The subject of the present invention is further described in more detail by reference to the following Figures. The abbreviations used in the Figures are explained in the subsequent description and in the Examples.
Figure 1 shows microbial colonies grown on a selective medium, wherein A represents growth without formation of a zone of clearance, and B represents growth with formation of a zone of clearance.
Figure 2 shows microbial growth and formation of a zone of clearance of isolate A2.
Figure 3 shows microbial growth and formation of a zone of clearance of isolate AnNf.
Figure 4 shows microbial growth and formation of a zone of clearance of isolate BTD6.
Figure 5 shows microbial growth and formation of a zone of clearance of isolate BTN1.
Figure 6 shows microbial growth and formation of a zone of clearance of isolate 15-2.
Figure 7 shows a graphical display (comparison) of the size of the zone of clearance (zone width in cm) achieved by active bacterial isolates on a selective medium.
Figure 8 shows microbial growth without formation of a zone of clearance of isolate A4.
Figure 9 shows a graphical display (comparison) of the chitinolytic activity of selected fungal isolates. EA: enzyme activity.
Figure 10 shows a graphical display (comparison) of the chitinolytic activity of selected bacterial isolates. EA: enzyme activity.
Figure 11 shows the dynamics of enzyme synthesis of fungal isolate H5 cultivated in a laboratory bioreactor.
Figure 12 shows the correlation between observed value and fitted value of chitinolytic activity expressed in U per ml of nutrient medium, obtained during optimization of the liquid nutrient medium.
Figure 13 shows the Estimated Response Surface of chitinolytic activity expressed in E per ml of nutrient medium, calculated during optimization of the liquid nutrient medium. LMW: low molecular weight chitosan; HMW: high molecular weight chitosan.
Figure 14 shows the standardized Pareto Chart of the standardized effect of the influence of different factors (concentration of low molecular weight (LMW) chitosan, high molecular weight (HMW) chitosan and colloidal chitin), calculated during optimization of the liquid nutrient medium. Legend see Table 5.
Figure 15 shows SEC after fractionation of strain H5 culture liquid by saturation with 60% (w/v) (NH₄)₂SO₄. Left y axis: Activity in AU/cm³; right y axis: Conductivity (indicating salt content) in mS/cm.
Figure 16 shows SEC after fractionation of strain H5 culture liquid by saturation with 60% (v/v) acetone. Legend: see Fig. 15.
Figure 17 shows SEC after fractionation of strain H5 culture liquid by saturation with 60% (v/v) isopropanol. Legend: see Fig. 15.
Figure 18 shows SEC after fractionation of strain H5 culture liquid by saturation with 70% (v/v) ethanol. Legend: see Fig. 15.
Figure 19 shows IEC after fractionation of strain H5 culture liquid by saturation with 60% acetone. Legend: see Fig. 15.
Figure 20 shows IEC after fractionation of strain H5 culture liquid by saturation with 60% (v/v) isopropanol. Legend: see Fig. 15.
Figure 21 shows IEC after fractionation of strain H5 culture liquid by saturation with 70% (v/v) ethanol. Legend: see Fig. 15.
Figure 22 displays the developed method for producing and purifying chitinase H5.
Figure 23 shows the Lineweaver-Burk plot for determining Kₘ and Vₘₐₓ of chitinase H5. 1/V: reciprocal conversion rate; 1/S: reciprocal substrate concentration.
Figure 24 shows the pH profile of unpurified chitinase H5.
Figure 25 shows the pH profile of partially purified chitinase H5.
Figure 26 shows the temperature profile ofunpurified chitinase H5.
Figure 27 shows the temperature profile of partially purified chitinase H5.

### DETAILED DESCRIPTION

The following terms and definitions will be used in the context of the present invention:
As used in the context of present invention, the singular forms of "a" and "an" also include the respective plurals unless the context clearly dictates otherwise. Thus, the term "a" or "an" is intended to mean "one or more" or "at least one", unless indicated otherwise.

The term "chitinase" in the context of the present invention refers to a hydrolytic enzyme which breaks down chitin.

The term "unpurified chitinase" in the context of the present invention refers to the culture supernatant of a chitinase producing strain, e.g. of strain H5, obtained after cultivation of said strain in liquid medium and subsequent separation of the culture medium from the strain cells.

The term "partially purified chitinase" in the context of the present invention refers to the fractions obtained after size-exclusion chromatography.

The term "zone of clearance" in the context of present invention refers to the clear area formed on a solid, chitin-containing nutrient medium outside of the growth zone of a microbial isolate. This clear area results from chitin hydrolysis and is an indicator for the chitinolytic activity of a microbial isolate.

The term "SEC" used in context of the present invention refers to size-exclusion chromatography. In the context of this invention, the term "SEC" in particular refers to size-exclusion chromatography of the enzyme chitinase H5. Depending on the context, "SEC" also refers to a size-exclusion chromatogram.

The term "IEC" used in context of the present invention refers to ion-exchange chromatography, or ion chromatography, which is a process that allows the separation of ions and polar molecules based on their charge. In the context of this invention, the term "IEC" in particular refers to ion-exchange chromatography of the enzyme chitinase H5. Depending on the context, "IEC" also refers to anion-exchange chromatogram.

The term "PCR" refers to polymerase chain reaction.

The term "GlcNAc" used in context of the present invention refers to *N-*acetylglucosamine, IUPAC name: 2-(acetylamino)-2-deoxy-D-glucose, synonym: NAG.

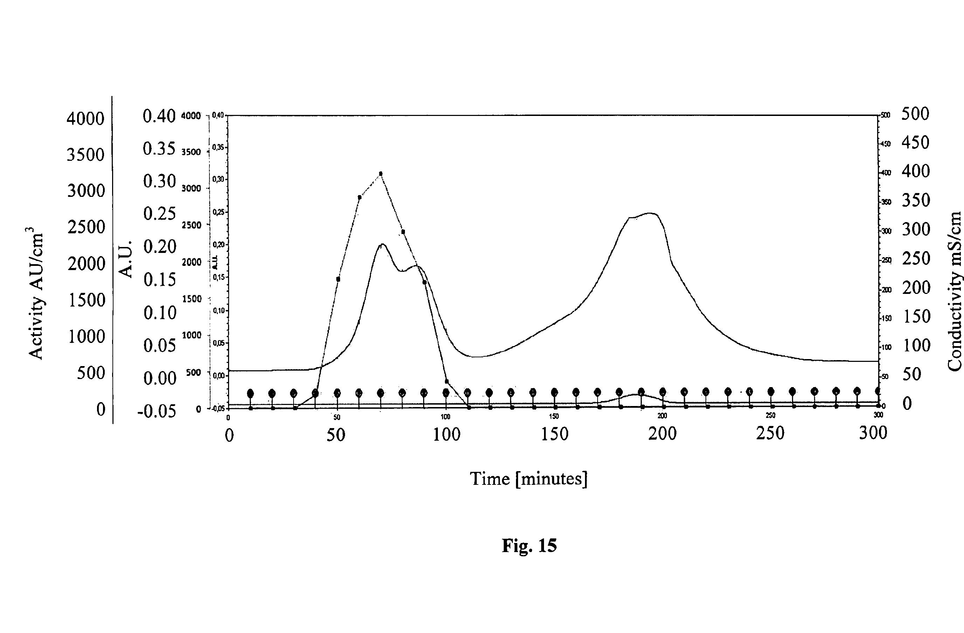

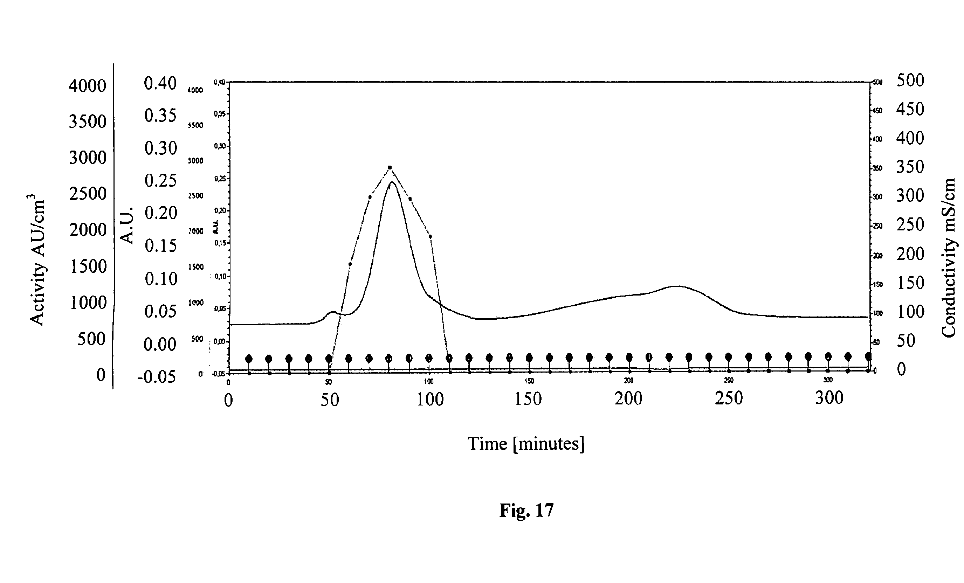

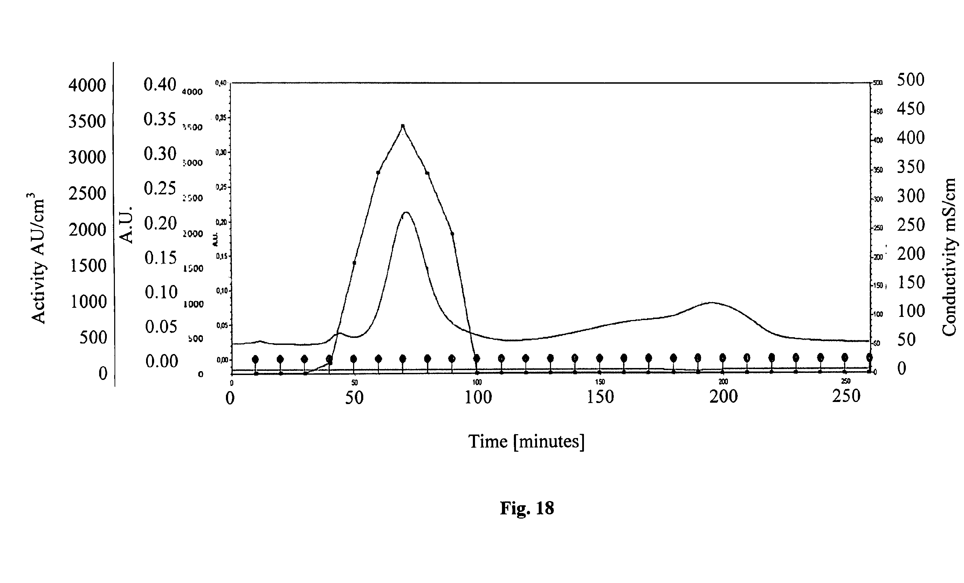

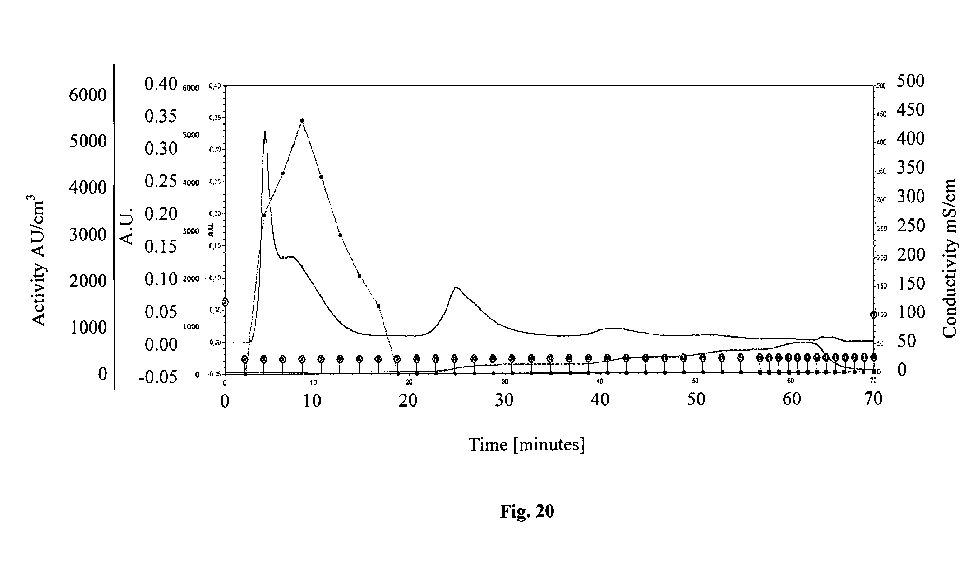

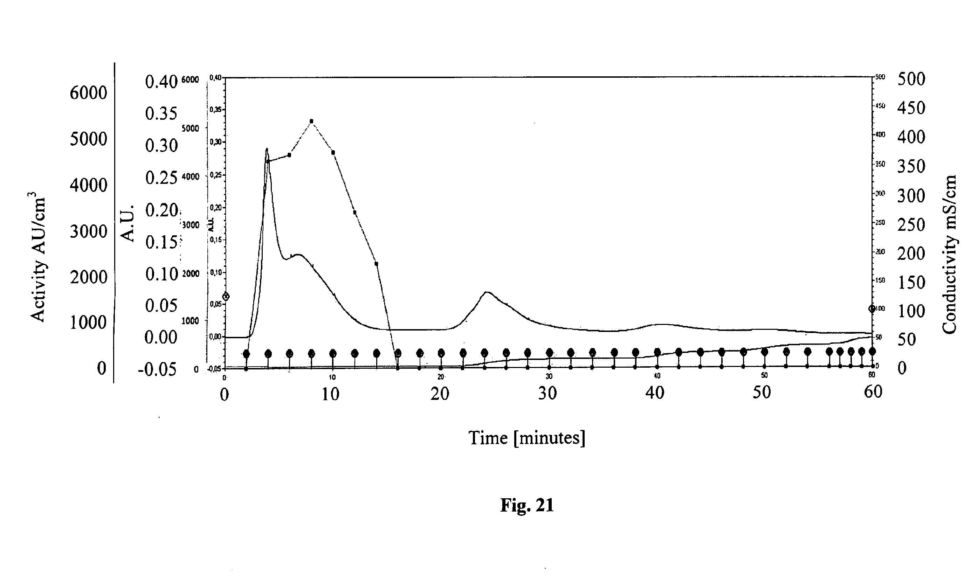

The terms "U", "E", and "AU" in the context of the present invention are all referring to the chitinolytic activity of chitinase. "U" and "E" are synonyms. One U is the amount of enzyme which catalyzes the production of 1 µmol *N*-acetyl-D-glucosamine per hour. In the context of the present invention, the chitinolytic activity may be measured based on the quantity of the reduced groups of chitin, which was determined by the addition of 3,5-dinitrosalicylic acid (DNS-reagent) (Miller G.L., (1959) Use of Dinitrosalicylic Acid Reagent for Determination of Reducing Sugar, Anal. Chem., Vol. 31, No. 3, 426-428). For measuring the chitinolytic activity, the following standard conditions may be used: 0.5% colloidal chitin as substrate is diluted in 0.1 M citric acid buffer having pH 6.0, and the enzymatic reaction is performed at 50°C for 1h. Subsequently, the quantity ofreduced groups of chitin is determined by addition of the DNS-reagent. The chitinolytic activity may also be measured by using the CM-Chitin-RBV method described in Example 8c at a standard temperature of 40°C. The CM-Chitin-RBV method is more sensitive and suitable for enzyme determination in very diluted solutions. For the CM-Chitin-RBV method, the chitinase activity is determined spectrophotometrically at 550 m, and the activity is expressed as absorption units (AU) per cm³ enzyme solution (as, e.g., in Figs. 15 to 21).

"NBIMCC 8735" is the access number of the *Aspergillus flavus* H5 strain of the present invention, deposited in the National Bank for Industrial Microorganisms and Cell Cultures (NBIMCC) - Bulgaria by Biodinamika Ltd., GK Trakia No. 398, entr. 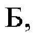 Plovdiv, 4000, Bulgaria.

The strains and enzymes of the present invention are preferably isolated. The term "isolated" indicates that an organism or enzyme named subsequent to said term, e.g. a strain or a chitinase of the present invention, has been removed from its native environment. E.g., the strain *Aspergillus flavus* H5 of the present invention has been isolated from its native environment soil, and the chitinase H5 has been isolated from its native environment which is said strain H5.

The present invention pertains to a novel *Aspergillus sp.* strain, wherein the strain is *Aspergillus flavus* H5, as deposited in the National Bank for Industrial Microorganisms and Cell Cultures (NBIMCC) - Bulgaria under the access no. NBIMCC 8735, and to a method for producing chitinase utilizing the same. Moreover, the present invention pertains to a chitinase, which will in the context of the present invention be designated as "chitinase H5", produced by said strain, to a use of said chitinase H5, and to a method and a cultivation medium for cultivating said strain H5.

One embodiment of the present invention is the *Aspergillus flavus* strain H5, deposited under the access number NBIMCC 8735.

A further embodiment of the present invention is a highly active chitinase H5, produced by the strain *Aspergillus flavus* H5.

Still a further embodiment of the present invention is a medium for culturing a strain producing a chitinase, for example the strain *Aspergillus flavus* H5. A method for culturing the strain is also an embodiment of the present invention.

Still a further embodiment of the present invention is a use of the chitinase H5 in a temporary or permanent anti-contamination coating system that can be applied to a surface for the detachment of chitin-containing debris (e.g., insect debris) from said surface, preferably from a flow surface (in particular a laminar flow surface), more preferably from an airfoil, a hydrofoil, or a blade of a wind energy conversion system, even more preferably from an airfoil, and most preferably from a laminar airfoil.

Still a further embodiment of the present invention is a use of a composition comprising the highly active chitinase H5 in a temporary or permanent anti-contamination coating system that can be applied to a surface for the detachment of chitin-containing debris (e.g., insect debris) from said surface, preferably from a flow surface (in particular a laminar flow surface), more preferably from an airfoil, a hydrofoil, or a blade of a wind energy conversion system, even more preferably from an airfoil, and most preferably from a laminar airfoil. In particular, still a further embodiment of the present invention is a use of the highly active chitinase H5 in a temporary cleaning composition for removing and/or preventing chitin-containing debris on said surfaces.

Still a further embodiment of the present invention is a coated surface, wherein the surface is coated with a composition comprising a chitinase, preferably the chitinase H5. The coated surface is preferably a flow surface (in particular a laminar flow surface), more preferably an airfoil, a hydrofoil, or a blade of a wind energy conversion system, even more preferably an airfoil, and most preferably a laminar airfoil.

It was now found that a novel microbial strain, which has been tentatively assigned to be an *Aspergillus sp.,* and is defined in the following as strain *Aspergillus flavus* H5, is able to produce a chitinase having a high chitinolytic activity. This strain, screened from other chitinase producing strains, was found to be an excellent producer of highly active chitinase, in the following defined as chitinase H5.

The chitinase produced by the strain *Aspergillus flavus* H5 according to the present invention is an extracellular chitinase. This is advantageous for purification of said chitinase from the strain.

Generally, enzymes involved in chitin hydrolysis may be divided into endochitinases and exochitinases. Endochitinase, 1,4-beta-poly-*N*-acetylglucosaminidase, catalyzes the hydrolysis of non-terminal β-1,4-linkages of *N*-acetyl-D-glucosamine (GlcNAc) polymers of chitin and chitodextrins. Exochitinase, β-*N*-acetylhexosaminidase, catalyzes the hydrolysis of the terminal β-1,4-linkages of *N*-acetyl-D-glucosamine (GlcNAc) polymers of chitin and chitodextrins. The chitinases obtained from fungi are mainly endochitinases, which can hydrolyze chitin into *N*-acetyl-D-glucosamine or other chitooligomers.

Moreover, chitinases are inducible enzymes, which means that chitin and its derivates have to be included in the nutrient medium for their biosynthesis. As inducers, glucosamine, *N*-acetyl-D-glucosamine, chitobiose and chitooligosaccharides can be applied. The choice of inducer depends on the strain characteristics. For example, the presence of *N*-acetyl-D-glucosamine represses the enzyme synthesis although the presence of chitobiose in the nutrient medium acts as an inducer of chitinase biosynthesis in the cultivation process of *serratia marcescens.*

Therefore, an optimal nutrient medium for selecting and for cultivating a chitinase producing strain is of great importance.

In general, a nutrient medium for selecting chitinase producing microorganisms comprises at least water, colloidal chitin as a sole carbon source, and minerals.

A selective nutrient medium used for bacteria selection may be composed of ammonium sulfate, potassium dihydrogen phosphate, dipotassium phosphate, magnesium sulfate heptahydrate, iron sulfate heptahydrate, calcium chloride dihydrate, colloidal chitin, and - if it is a solid medium - Agar-agar.

A selective nutrient medium used for fungi selection may be composed of potassium dihydrogen phosphate, dipotassium phosphate, sodium chloride, magnesium sulfate heptahydrate, colloidal chitin, and - if it is a solid medium - Agar-agar.

The capability of growth and formation of a zone of clearance, resulting from chitin hydrolysis, can be observed in the agar media. Only microorganisms showing intensive growth or formation of a zone of clearance were chosen for further analyses in the context of the present invention (see Examples 1 to 3).

In order to characterize a strain which produces chitinase, measurement of chitinolytic activity has to be performed. In the context of the present invention, the chitinolytic activity may be measured as described in Example 3.

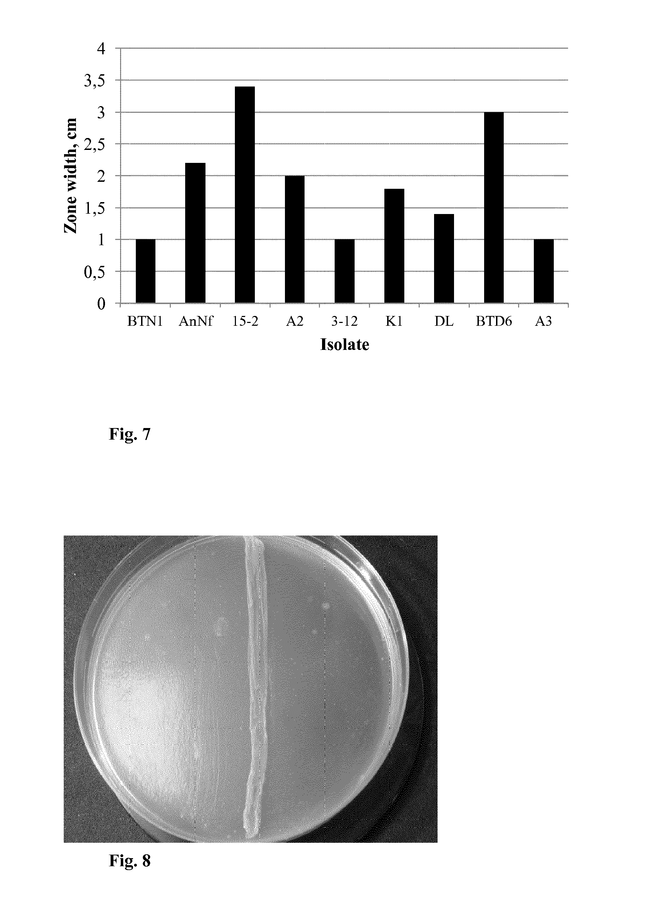

It was found that of the fungal isolates, isolates H5, CH2 and CHM1 showed the highest chitinolytic activities, and of the bacterial isolates, isolates AnNf, 15-2 and BTD6, as shown in Figures 7 and 8. Isolate H5, also named strain H5, showing the highest chitinolytic activity of all selected isolates was further investigated as described in Example 4.

It was found that under the conditions of Example 4 the enzyme activity increased between 24 h and 120 h and was highest between 72 h and 96 h.

The new strain was tentatively taxonomically identified as *Aspergillus flavus* as described in Example 5.

The identified strain, showing a high chitinolytic activity, was designated as *"Aspergillus flavus* H5". The newly identified strain *Aspergillus flavus* H5 is deposited in the National Bank for Industrial Microorganisms and Cell Cultures (NBIMCC) - Bulgaria under the access no. NBIMCC 8735.

This strain is one embodiment of the present invention.

The chitinase produced by said strain which is defined as chitinase H5 in the context of the present invention is a further embodiment of the present invention.

In order to synthesize higher amounts of chitinase H5 produced by *Aspergillus flavus* H5 of to the present invention, the nutrient medium for the strain *Aspergillus flavus* H5 was optimized, taking into account that the chitinase biosynthesis is induced by its substrate chitin (see Example 6). The following optimal medium was found: peptone 3.0 g/l, glucose 1.0 g/l, K₂HPO₄ 0.2 g/l, yeast extract 2.0 g/l, MgSO₄*7H₂O 0.1 g/l, colloidal chitin 5.96 g/l, low molecular weight chitosan 10.08 g/l, and high molecular weight chitosan 0.12 g/l.

It was found that a cultivation *o*f *Aspergillus flavus* H5 in the above-mentioned medium at 28°C with continuous stirring and aeration after 96 h, resulted in an enzyme activity of the culture medium of 6845.24 AU/cm³.

Thus, the medium of the present invention for culturing a chitinase-producing microbial strain, preferably a fungal strain, in particular the strain *Aspergillus flavus* H5 of the present invention, and thus producing chitinase comprises the following components: colloidal chitin, low molecular weight chitosan, and high molecular weight chitosan.

The medium preferably comprises colloidal chitin prepared as described in Example 1 from chitin from crab shell (e.g., Sigma - 7170, CAS No.: 1398-61-4, practical grade, powder form).

Chitosan is commercially available in several different forms. Typically, low molecular weight (150,000) chitosan is 75-85% deacetylated and has a viscosity of 20-200 cps (Brookfield, 1% solution in 1% acetic acid). The medium according to the present invention preferably comprises low molecular weight chitosan with a viscosity of from 100 cps to 200 cps, more preferably of from 150 to 200 cps, and even more preferably of about 200 cps. Typically, high molecular weight (600,000) chitosan is a coarsely ground polymer prepared from crab or shrimp shell and has a viscosity of 400-2000 cps. The medium according to the present invention preferably comprises high molecular weight chitosan with a viscosity of at least 400 cps.

In one embodiment of the present invention, the medium additionally comprises:
(i) a nitrogen-source,
(ii) an additional carbon-source, and
(iii) one or more mineral salts.

In a preferred embodiment of the present invention, the medium comprises the following components: colloidal chitin, low molecular weight chitosan, high molecular weight chitosan, and one or more of the following components: peptone, glucose, K₂HPO₄, yeast extract, and MgSO₄*7H₂O.

In a more preferred embodiment of the present invention, the medium comprises or consists of the following components: peptone, glucose, K₂HPO₄, yeast extract, MgSO₄*7H₂O, colloidal chitin, low molecular weight chitosan, high molecular weight chitosan, and water.

The medium preferably comprises colloidal chitin in an amount of from 4.0 to 8.0 g/l, low molecular weight chitosan in an amount of from 8.0 to 12.0 g/l, and high molecular weight chitosan in an amount of from 0.08 to 0.4 g/l, more preferably colloidal chitin in an amount of from 5.0 to 7.0 g/l, low molecular weight chitosan in an amount of from 9.0 to 11.0 g/l, and high molecular weight chitosan in an amount of from 0.09 to 0.25 g/l, still more preferably colloidal chitin in an amount of from 5.5 to 6.5 g/l, low molecular weight chitosan in an amount of from 9.75 to 10.35 g/l, and high molecular weight chitosan in an amount of from 0.095 to 0.19 g/l, still more preferably colloidal chitin in an amount of from 5.9 to 6.0 g/l, low molecular weight chitosan in an amount of from 10.0 to 10.16 g/l, and high molecular weight chitosan in an amount of from 0.1 to 0.14 g/l, and most preferably colloidal chitin in an amount of 5.96 g/l, low molecular weight chitosan in an amount of 10.08 g/l, and high molecular weight chitosan in an amount of 0.12 g/l.

The medium preferably comprises peptone in an amount of from 2.0 to 4.0 g/l, more preferably in an amount of from 2.5 to 3.5 g/l, still more preferably in an amount of from 2.75 to 3.25 g/l, still more preferably in an amount of from 2.9 to 3.1 g/l, and most preferably in an amount of 3.0 g/l.

The medium preferably comprises glucose in an amount of from 0 to 1.8 g/l, more preferably in an amount of from 0.5 to 1.5 g/l, still more preferably in an amount of from 0.75 to 1.25 g/l, still more preferably in an amount of from 0.9 to 1.1 g/l, and most preferably in an amount of 1.0 g/l.

The medium preferably comprises K₂HPO₄ in an amount of from 0.1 to 0.3 g/l, more preferably in an amount of from 0.15 to 0.25 g/l, still more preferably in an amount of from 0.175 to 0.225 g/l, still more preferably in an amount of from 0.19 to 0.21 g/l, and most preferably in an amount of 0.2 g/l.

The medium preferably comprises yeast extract in an amount of from 0 to 3.0 g/l, more preferably in an amount of from 1.0 to 2.5 g/l, still more preferably in an amount of from 1.75 to 2.25 g/l, still more preferably in an amount of from 1.9 to 2.1 g/l, and most preferably in an amount of 2.0 g/l.

The medium preferably comprises MgSO₄*7H₂O in an amount of from 0.02 to 0.18 g/l, more preferably in an amount of from 0.05 to 0.15 g/l, still more preferably in an amount of from 0.075 to 0.125 g/l, still more preferably in an amount of from 0.09 to 0.11 g/l, and most preferably in an amount of 0.1 g/l.

In a particularly preferred embodiment, the medium of the present invention comprises the following components: peptone 3.0 g/l, glucose 1.0 g/l, K₂HPO₄ 0.2 g/l, yeast extract 2.0 g/l, MgSO₄*7H₂O 0.1 g/l, colloidal chitin 5.96 g/l, low molecular weight chitosan 10.08 g/l, and high molecular weight chitosan 0.12 g/l.

This liquid nutrient medium is used in one embodiment in a method for producing a chitinase, preferably an extracellular chitinase, more preferably the chitinase H5 of the present invention.

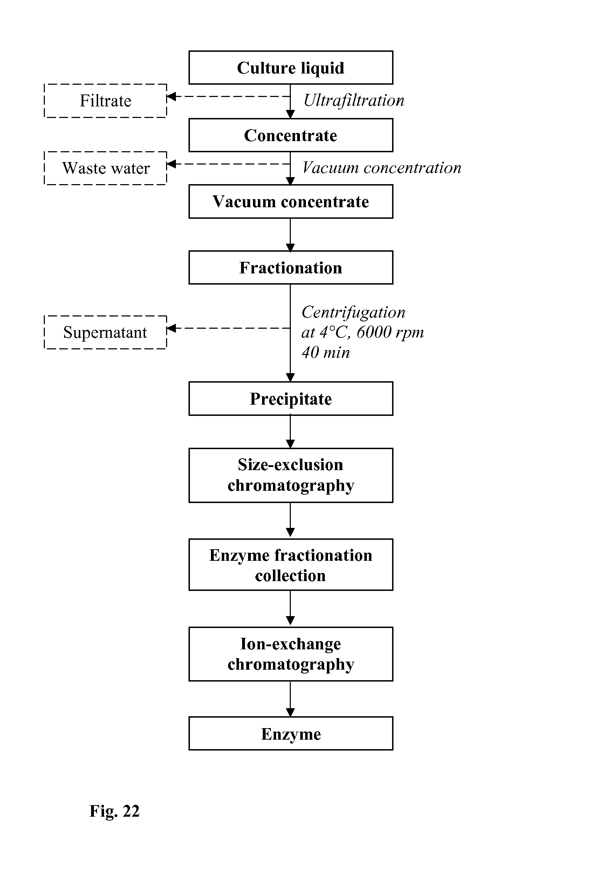

After growth of a chitinase-producing microbial strain, e.g., the strain *Aspergillus flavus* H5, in the above-described optimal liquid nutrient medium, the chitinase, e.g., the chitinase H5, can be purified and concentrated. A method for purification of the chitinase is a further embodiment of the present invention. It was found that employing the steps depicted in Figure 22 yields the chitinase H5 of the present invention in a partially purified form, which can hence be produced at low cost and is highly active.

Initial enzyme purification and concentration can be accomplished by means of standard ultrafiltration and vacuum concentration procedures known to those skilled in the art. It was found that using these procedures a 5 fold increased enzyme activity was achieved with chitinase H5.

Fractionation or precipitation can be used for further purification of the chitinase. In one embodiment, salting-out is used to precipitate the chitinase. For salting-out, any method known in the art can be used, preferably a method which does not impair the activity of the chitinase. Addition of ammonium sulfate is used as one preferred method of fractionation in the context of the present invention.

The addition of organic solvents, such as ethanol, 2-propanol and acetone is used as another preferred method of fractionation to cause the chitinase in solution to precipitate. In a preferred embodiment, the fractionation is performed by addition of an organic solvent, preferably an organic solvent miscible with water, more preferably an organic solvent selected from the group consisting of methanol, ethanol, propanol, isopropanol, acetonitrile, acetone, butanediol, dimethylformamid, DMSO, dioxane, ethylamine, ethylenglycol, glycerol, propanediol, pyridine , THF, and mixtures thereof, preferably by the addition of an organic solvent selected from the group consisting of methanol, ethanol, propanol, isopropanol, acetonitrile, acetone, dimethylformamid, DMSO, dioxane, THF, and mixtures thereof, more preferably by the addition of an organic solvent selected from the group consisting of methanol, ethanol, propanol, isopropanol, acetone and mixtures thereof, and most preferably by the addition of ethanol, acetone or isopropanol, or mixtures thereof, and most preferably by the addition of ethanol.

Important parameters to consider for fractionation are temperature, pH and protein concentration in solution. These considerations are within the ambit of the knowledge of a person skilled in the art of protein isolation. The temperature for fractionation is preferably from 0 to 10°C, more preferably from 1 to 8°C, even more preferably from 2 to 6°C and most preferably from 2 to 4°C.

Preferably, the fractionation is performed in at least two steps with different fractionation conditions (stepwise fractionation). In a first step, ballast protein may be removed. In a second or subsequent step, the chitinase may be precipitated from the supernatant of said first step. Thus, a simple purification of the chitinase from ballast protein can be achieved. Stepwise fractionation may comprise (i) in a first step, addition of preferably 20-40 % (v/v), more preferably 25-35% (v/v), still more preferably 28-32% (v/v), and most preferably 30% (v/v) organic solvent to precipitate ballast proteins, and
(ii) in a second step, addition of preferably 70-90 % (v/v), more preferably 75-85%
(v/v), still more preferably 78-82% (v/v), and most preferably 80% (v/v) organic solvent to the supernatant to precipitate the enzyme.

In said stepwise fractionation, the organic solvent is preferably an organic solvent as described above. In each step, the same or different organic solvent may be used.

In a specific aspect, the stepwise fractionation may comprise
(i) in a first step, addition of preferably 20-40 % (v/v), more preferably 25-35% (v/v), still more preferably 28-32% (v/v), and most preferably 30% (v/v) ethanol to precipitate ballast proteins, and
(ii) in a second step, addition of preferably 70-90 % (v/v), more preferably 75-85% (v/v), still more preferably 78-82% (v/v), and most preferably 80% (v/v) ethanol to the supernatant to precipitate the enzyme.

The precipitate resulting from fractionation may then be purified additionally by means of size-exclusion chromatography and/or ion-exchange chromatography.

In a preferred method for purifying chitinase, in particular the chitinase H5 according to the present invention, sample separation by means of size-exclusion chromatography follows after fractionation. Size-exclusion chromatography may be performed by using 0.05 M sodium chloride as the mobile phase and a polyacrylamide gel matrix with a particle size of 90-180 µm ("Bio-Gel^{®} P-100 Gel", Bio-Rad) as the stationary phase. Figures 15-18 display size-exclusion chromatograms after fractionation of chitinase H5.

The peak possessing a chitinolytic activity obtained after size-exclusion chromatography of a sample previously fractionated with ammonium sulfate (Figure 15) was not well-defined and clearly separated.

Hence, in a preferred embodiment of the purification method of the present invention, the fractionation is performed by the addition of ethanol, acetone or isopropanol, or mixtures thereof, and most preferably by the addition of ethanol.

In the purification method according to the present invention, the fractions which are obtained by size-exclusion chromatography may further be processed by ion-exchange chromatography. Alternatively, IEC may be used instead of SEC.

In a preferred embodiment, the purification method comprises IEC as one of its steps, and more preferably in addition an SEC step which advantageously precedes the IEC step. The IEC may be performed as described in Example 7c.

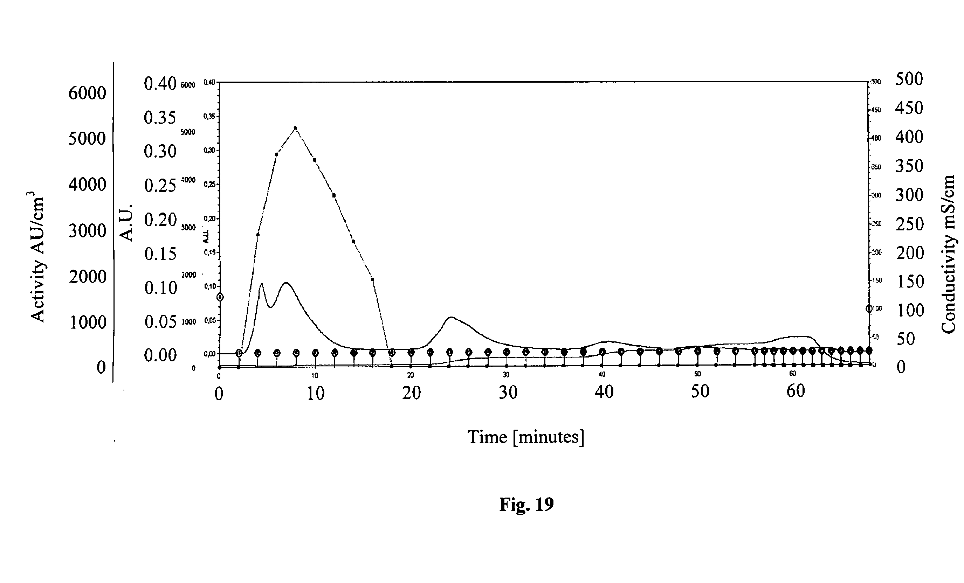

The ion-exchange chromatograms of Example 7c showed that solvents (alcohols and acetone) precipitate the chitinase in a very similar way (Figures19-21). The enzyme activity was determined in the first peaks that passed through the ion-exchange column, which means that the chitinase H5 is either positive charged or is a neutral protein.

In one embodiment of the present invention, the strain *Aspergillus flavus* H5 according to the present invention is used for the production of chitinase, preferably of extracellular chitinase, in particular of chitinase H5.

In another embodiment, a chitinase-producing strain, preferably the strain *Aspergillus flavus* H5 of the present invention, is used in a method for producing a chitinase, preferably an extracellular chitinase, in particular the chitinase H5 of the present invention.

The method for producing a chitinase, in particular the chitinase of the present invention from the strain *Aspergillus flavus* H5 of the present invention, comprises the following steps:
a) culturing a chitinase-producing strain, preferably the strain *Aspergillus flavus* H5 of the present invention, in a liquid nutrient medium, preferably in the liquid nutrient medium of the present invention;
b) separating the nutrient medium from the strain cells, e.g. by centrifugation, thus retrieving the cultivation supernatant; and
c) isolating the chitinase from the cultivation supernatant, preferably *via* one or more of the purification steps described above.

In a particularly preferred embodiment of the method for producing a chitinase of the present invention, step (a) comprises culturing the chitinase-producing strain in a liquid nutrient medium comprising the following components: peptone 3.0 g/l, glucose 1.0 g/l, K₂HPO₄ 0.2 g/l, yeast extract 2.0 g/l, MgSO₄*7H₂O 0.1 g/l, colloidal chitin 5.96 g/l, low molecular weight chitosan 10.08 g/l, and high molecular weight chitosan 0.12 g/l.

In a particularly preferred embodiment of the method for producing a chitinase of the present invention, step (c) encompasses a fractionation step as described above.

In an especially preferred embodiment, the chitinase-producing strain is the *Aspergillus flavus* H5 of the present invention, step (a) consists of culturing the chitinase-producing strain in a liquid nutrient medium comprising the following components: peptone 3.0 g/l, glucose 1.0 g/l, K₂HPO₄ 0.2 g/l, yeast extract 2.0 g/l, MgSO₄*7H₂O 0.1 g/l, colloidal chitin 5.96 g/l, low molecular weight chitosan 10.08 g/l, and high molecular weight chitosan 0.12 g/l, and step (c) encompasses all of the purification steps of the purification method as described above.

In order to characterize the enzyme chitinase H5, kinetic constants of partially purified chitinase H5 were determined in the Examples using standard techniques known to those skilled in the art.

Two of the most important parameters for enzymatic reactions are Vₘₐₓ and Kₘ. The parameter Vₘₐₓ represents the maximum conversion rate achieved by the enzyme, at maximum (saturating) substrate concentrations. The Michaelis constant Kₘ is the substrate concentration at which the reaction rate is half of Vₘₐₓ. In other words, Kₘ and Vₘₐₓ are the two parameters which define the kinetic behavior of an enzyme as a function of the substrate.

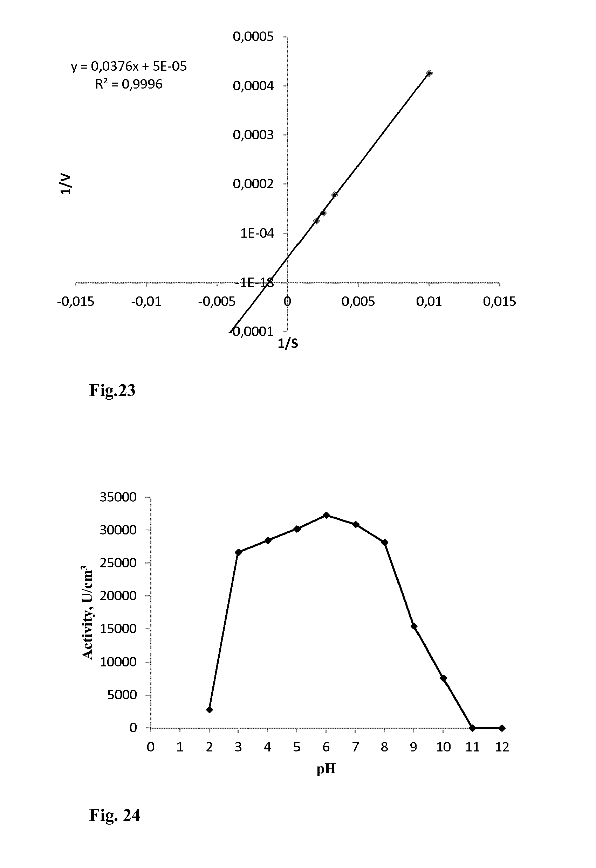

Kₘ and Vₘₐₓ are determined using standard procedures known to those skilled in the art. In an exemplary embodiment, in order to determine Kₘ and Vₘₐₓ, different concentrations of carboxymethyl-chitin-remazol-brilliant-violet (CM-Chitin-RBV, Loewe Biochemica GmbH, Germany) are used: 0.04, 0.08, 0.12, 0.16 and 0.2 mg/cm³. During this observation the following conditions were applied: 40°C for 10 min at pH 8.0, which may be applied as standard conditions for determining Kₘ and Vₘₐₓ in the context of the present invention. The results obtained for the chitinase H5 were: Kₘ=752 µg/cm³; Vₘₐₓ=20000 AU/min, which indicates a good affinity between the enzyme chitinase H5 and the substrate. Figure 23 shows a Lineweaver-Burk plot for determining Kₘ and Vₘₐₓ of chitinase H5.

Thus, the chitinase of the present invention preferably has a Kₘ in the range of from 640 to 864 µg/cm³, more preferably in the range of from 676 to 828 µg/cm³, still more preferably in the range of from 714 to 790 µg/cm³, still more preferably in the range of from 746 to 758 µg/cm³, and most preferably of 752 µg/cm³.

The chitinase of the present invention preferably has a Vₘₐₓ in the range of from 17000 to 23000 AU/min, more preferably in the range of from 18000 to 22000 AU/min, still more preferably in the range of from 19000 to 21000 AU/min, still more preferably in the range of from 19800 to 20200 AU/min, and most preferably of 20000 AU/min.

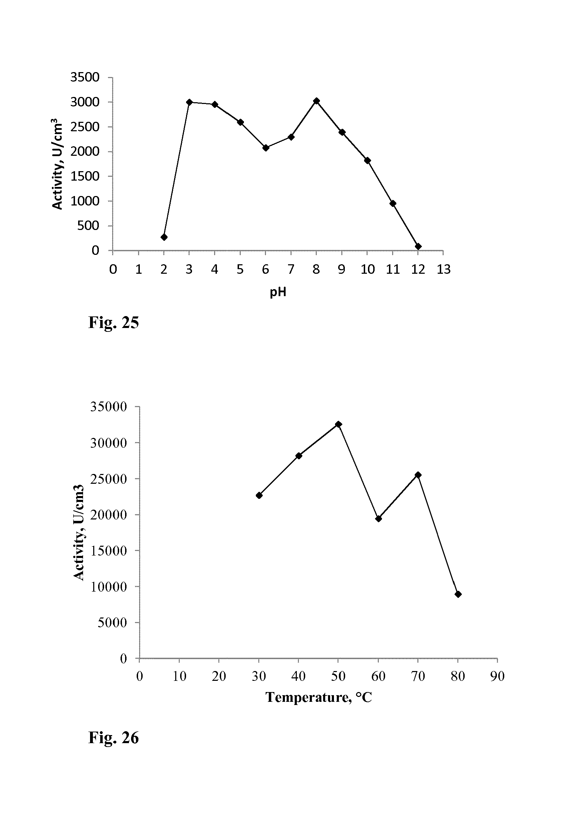

One parameter characterizing the chitinase of the present invention is its pH profile. Chitinase H5 has two clearly defined optimum pH values: one at pH 3.0 and one at pH 8.0 (Figure 25). In addition, chitinase H5 maintains its activity in a very wide pH range, i.e. from 3.0 to 8.0 and its activity varies at a very low level.

The possession of two optimum pH values is an advantage of this enzyme over chitinases of the prior art. The two optimum pH values make this enzyme applicable in different industries for different purposes using alkaline as well as acidic media.

Thus, the chitinase of the present invention preferably has a first pH optimum in a pH range of from 1.0 to 5.0, and a second pH optimum in a pH range of from 7.0 to 10.0, more preferably a first pH optimum in a pH range of from 2.0 to 4.0, and a second pH optimum in a pH range of from 7.5 to 9.0, still more preferably a first pH optimum in a pH range of from 2.5 to 3.5, and a second pH optimum in a pH range of from 7.5 to 8.5, and most preferably a first pH optimum at 3.0, and a second pH optimum at 8.0.

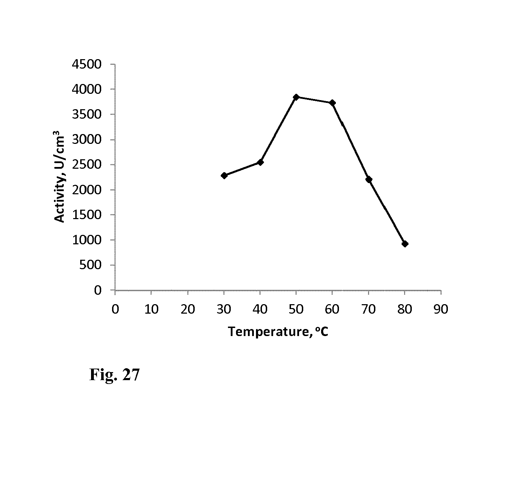

The temperature may also have a significant influence on the enzyme action. The chitinolytic activity of the chitinase according to the present invention was studied in the range of from 30°C to 80°C. It has been found that the chitinase H5 of the present invention has a maximal enzyme activity at 50°C (Figures 26 and 27). Therefore, the strain *Aspergillus flavus* H5 of the present invention produces a high-temperature resistant chitinase, which is advantageous for its application in technical environments requiring a high temperature, e.g. in the fields of medicine, agriculture, biotechnology, waste management and industry, in particular in the field of and aerodynamics.

Thus, the chitinase of the present invention preferably has a Tₘₐₓ in the range of from 30 to 70°C, more preferably in the range of from 40 to 60°C, still more preferably in the range of from 45 to 55°C, still more preferably in the range of from 47.5 to 52.5°C, and most preferably of 50°C.

In a preferred embodiment, the chitinase of the present invention preferably has a Kₘ in the range of from 640 to 864 µg/cm³, a first pH optimum in a pH range of from 1.0 to 5.0, and a second pH optimum in a pH range of from 7.0 to 10.0, more preferably a Kₘ in the range of from 676 to 828 µg/cm³, a first pH optimum in a pH range of from 2.0 to 4.0, and a second pH optimum in a pH range of from 7.5 to 9.0, still more preferably Kₘ in the range of from 714 to 790 µg/cm³, a first pH optimum in a pH range of from 2.5 to 3.5, and a second pH optimum in a pH range of from 7.5 to 8.5, and most preferably a Kₘ of 752 µg/cm³, a first pH optimum at 3.0, and a second pH optimum at 8.0.

In a more preferred embodiment, the chitinase of the present invention preferably has a Tₘₐₓ in the range of from 30 to 70°C, a Kₘ in the range of from 640 to 864 µg/cm³, a first pH optimum in a pH range of from 1.0 to 5.0, and a second pH optimum in a pH range of from 7.0 to 10.0, more preferably a Tₘₐₓ in the range of from 40 to 60°C, a Kₘ in the range of from 640 to 864 µg/cm³, a first pH optimum in a pH range of from 1.0 to 5.0, and a second pH optimum in a pH range of from 7.0 to 10.0, more preferably a Tₘₐₓ in the range of from 45 to 55°C, a Kₘ in the range of from 676 to 828 µg/cm³, a first pH optimum in a pH range of from 2.0 to 4.0, and a second pH optimum in a pH range of from 7.5 to 9.0, still more preferably a Tₘₐₓ in the range of from 47.5 to 52.5°C, a Kₘ in the range of from 714 to 790 µg/cm³, a first pH optimum in a pH range of from 2.5 to 3.5, and a second pH optimum in a pH range of from 7.5 to 8.5, and most preferably a Tₘₐₓ of 50°C, a Kₘ of 752 µg/cm³, a first pH optimum at 3.0, and a second pH optimum at 8.0.

In a particularly preferred embodiment, the chitinase according to the present invention is characterized by a Kₘ of 752 µg/cm³, and two optimum pH values at 3.0 and 8.0.

In another particularly preferred embodiment, the chitinase according to the present invention is characterized by a Kₘ of 752 µg/cm³, a Vₘₐₓ of 20000 AU/min, a maximal enzymatic activity at 50°C, and two optimum pH values at 3.0 and 8.0.

Having characterized the chitinase according to the present invention, its application and use, as well as methods for its use shall be briefly described.

In one embodiment, the chitinase according to the present invention is used in a method for degrading chitin. The method for degrading chitin comprises hydrolyzing chitin using a chitinase, preferably an extracellular chitinase, of the present invention, in particular the chitinase H5 of the present invention.

Hydrolyzation of chitin is preferably performed at a temperature in the range of from 30 to 70°C, more preferably from 40 to 60°C, still more preferably from 45 to 55°C, still more preferably from 47.5 to 52.5°C, and most preferably at a temperature of 50°C.

Hydrolyzation of chitin may preferably be performed at a pH in the range of from 1.0 to 5.0, more preferably from 2.0 to 4.0, still more preferably from 2.5 to 3.5, and most preferably at a pH of 3.0.

Hydrolyzing chitin may also preferably be performed at a pH in the range of from 7.0 to 10.0, more preferably from 7.5 to 9.0, still more preferably from 7.5 to 8.5, and most preferably at a pH of 8.0.

The chitinase of the present invention may be used in many technical fields, such as medicine, agriculture, biotechnology, waste management and industry. The chitinase may be used for the preparation of GlcNAc and as a safe biocontrol agent.

In particular, the chitinase of the present invention may be used in the field of aerodynamics or hydrodynamics. E.g., the chitinase of the present invention may be used for preventing and/or removing of insect debris from wing, fuselage and/or tail of an aircraft.

In an aspect of the present invention, the chitinase is used in a temporary or permanent anti-contamination coating system. Preferably, the temporary or permanent anti-contamination coating system is applied to a surface for the detachment of chitin-containing debris (e.g., insect debris) from said surface. More preferably, the surface is a flow surface (in particular a laminar flow surface), still more preferably an airfoil, a hydrofoil, or a blade of a wind energy conversion system, even more preferably an airfoil, and most preferably a laminar airfoil. Most preferably, in one aspect of the present invention the chitinase is used in a temporary cleaning composition for removing and/or preventing chitin-containing debris on any of said surfaces, in particular on an airfoil.

By applying the chitinase, which decomposes chitin, the insect deposits on surfaces of aircrafts are removed, thereby resulting in a reduction of turbulent flows on the aircraft surface, in less air resistance and thus lower fuel consumption. The same consideration applies, *mutatis mutandis,* to other (laminar) flow surfaces.

The chitinase of the present invention may be used in a composition. A composition comprising the chitinase may be used for a temporary or permanent anti-contamination coating system. In particular, the composition comprising the chitinase may be used as a temporary cleaning composition for removing and/or preventing chitin-containing debris on a surface. Preferably, the temporary or permanent anti-contamination coating system may be applied to a surface for the detachment of chitin-containing debris (e.g., insect debris) from said surface. More preferably, the surface may be a flow surface (in particular a laminar flow surface), still more preferably an airfoil, a hydrofoil, or a blade of a wind energy conversion system, even more preferably an airfoil, and most preferably a laminar airfoil.

The composition may comprise further additive components such as further enzymes, surfactants, de-icing components and thickening agents.

In another aspect of the present invention, the chitinase is used in a method for temporary or permanent coating of a surface for the detachment of chitin-containing debris (e.g., insect debris) from said surface. More preferably, the surface may be a flow surface (in particular a laminar flow surface), still more preferably an airfoil, a hydrofoil, or a blade of a wind energy conversion system, even more preferably an airfoil, and most preferably a laminar airfoil. In a preferred embodiment, the chitinase is used in a method for temporary cleaning a surface to remove and/or prevent chitin-containing debris on said surface.

The above-described strain of *Aspergillus flavus* H5 allows for a low cost production of highly active chitinase H5.

The present invention is further described in more detail by reference to the following examples. It should be understood that these examples are for illustrative purposes only and not to be construed as limiting the invention.

### EXAMPLES

### Example 1: Screening for microorganisms which produce chitinolytic enzymes

The primary screening for microorganisms which produce chitinolytic enzymes was started with different bacterial and fungal isolates from soil. The microorganisms were compared in their capability to degrade colloidal chitin on an agar nutrient medium, and in their capability to synthesize chitinolytic enzymes in a liquid nutrient medium. The selection was performed on selective nutrient media containing colloidal chitin as a sole carbon source (Table 1 and Table 2).

**Table 1: The following agar nutrient media were used (amounts in g/l):**

| For bacteria selection: | | | For fungi selection: | | |
|---|---|---|---|---|---|
| 1. | (NH₄)₂SO₄ | 1.0 | 1. | KH₂PO₄ | 0.7 |
| 2. | KH₂PO₄ | 0.2 | 2. | K₂HPO₄ | 0.3 |
| 3. | K₂HPO₄ | 1.6 | 3. | NaCl | 2.0 |
| 4. | MgSO₄*7H₂O | 0.2 | 4. | MgSO₄*7H₂O | 0.5 |
| 5. | FeSO₄*7H₂O | 0.01 | 5. | colloidal chitin | 5.0 |
| 6. | CaCl₂*2H₂O | 0.02 | 6. | Agar-agar | 20.0 |
| 7. | colloidal chitin | 5.0 | | | |
| 8. | Agar-agar | 20.0 | | | |

**Table 2: The following liquid nutrient media were used (amounts in g/l):**

| For bacteria selection: | | | For fungi selection: | | |
|---|---|---|---|---|---|
| 1. | (NH₄)₂SO₄ | 1.0 | 1. | KH₂PO₄ | 0.7 |
| 2. | KH₂PO₄ | 0.2 | 2. | K₂HPO₄ | 0.3 |
| 3. | K₂HPO₄ | 1.6 | 3. | NaCl | 2.0 |
| 4. | MgSO₄*7H₂O | 0.2 | 4. | MgSO₄*7H₂O | 0.5 |
| 5. | FeSO₄*7H₂O | 0.01 | 5. | colloidal chitin | 5.0 |
| 6. | CaCl₂*2H₂O | 0.02 | 6. | high molecular weight chitosan (min 400 cps) | 5.0 |
| 7. | colloidal chitin | 5.0 | | | |
| 8. | high molecular weight chitosan (min 400 cps) | 5.0 | | | |

Colloidal chitin was prepared according to the method of Saadoun et al., (2009) Influence of culture conditions of Streptomyces sp. (Strain S242) on chitinase production. Polish Journal of Microbiology, Vol.58, No. 4, 339-345.200 ml concentrated HCl (Merck, 1.00317) was added to 20 g chitin (Sigma, C7177, CAS No.: 1398-61-4). The mixture was stirred periodically for 1 hour. 11 of cooled water (4-6°C) was poured into the mixture. After 10 min of stirring, the mixture was vacuum filtered through filter paper (Advantec 5B - 125mm, pore size 5-10 µm). The filtered chitin was re-suspended within 11 of water and filtered again. This procedure was repeated until a pH value of not less than 4.0 of the washing water was achieved.

The media were sterilized in an autoclave for 20 min. The sterile agar media were poured into Petri dishes. 0.1 ml of the pre-prepared microorganism suspensions were spread onto the Petri dishes. The fungal and bacterial isolates were grown at 28°C for 20 days.

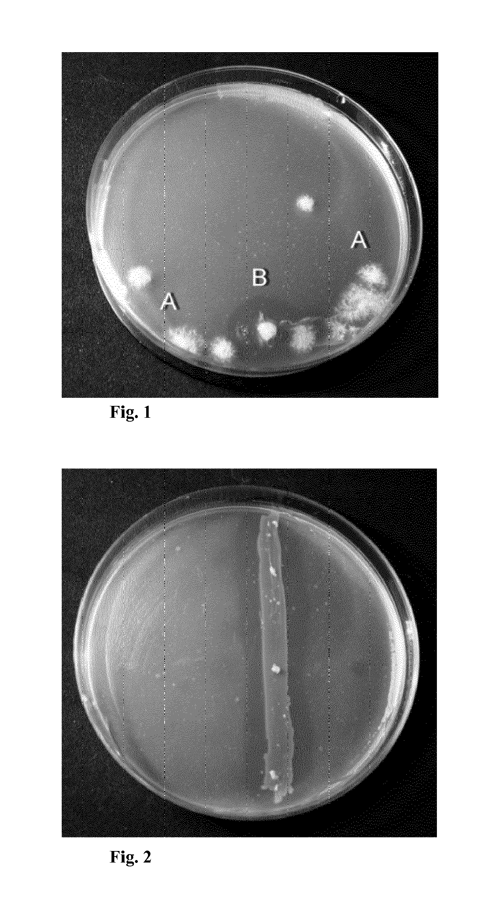

The capability of growth and formation of a zone of clearance, resulting from chitin hydrolysis, were observed in the media. Only microorganisms showing intensive growth or formation of a zone of clearance (Fig. 1) were chosen for further analyses. Fig. 1 shows microbial colonies grown on a selective media of Table 1, wherein A represents growth without formation of a zone of clearance and B represents growth with formation of a zone of clearance.

### Example 2: Streaking of the pre-selected microorganisms which produce chitinolytic enzymes

Streaking of the bacterial and fungal microorganisms pre-selected in Example 1 was performed for further selection on the same selective media. The pre-selected fungal and bacterial isolates were grown at 28°C for 20 days.

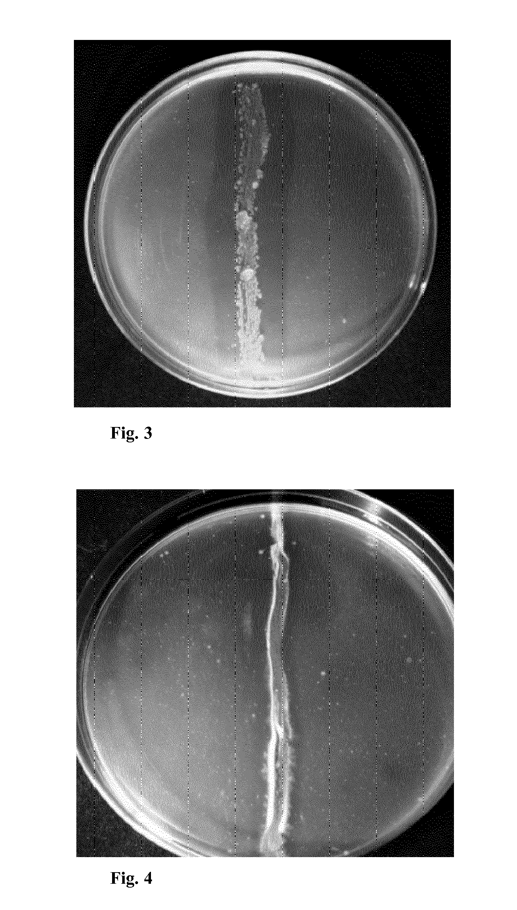

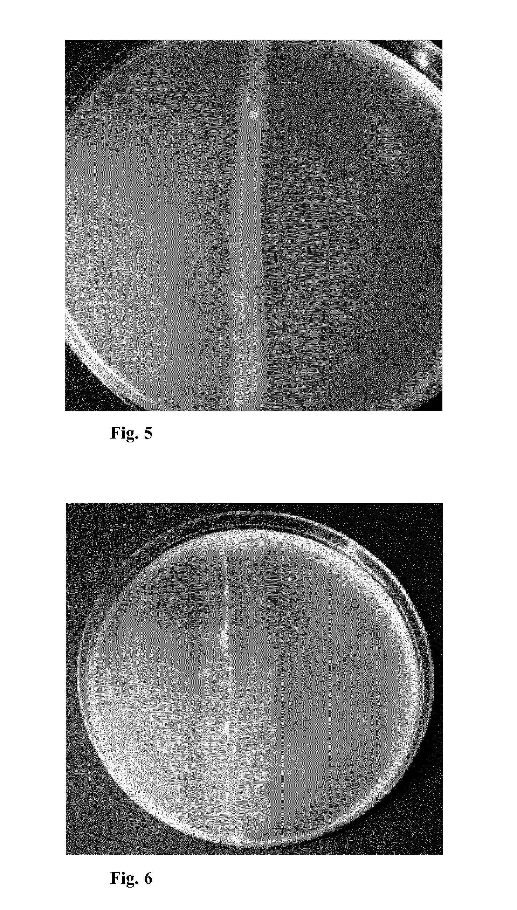

The capability of growth and formation of a zone of clearance, resulting from chitin hydrolysis, was observed in the media. The zone of clearance, due to chitin hydrolysis, differed in size and in form of the border part. Isolates BTN1, AnNf, 15-2, A2, 3-12, K1, DL and A3 were able to form a clearly defined zone of clearance, caused by almost full chitin degradation. Fig. 2 and 3 shows exemplary growth and formation of a zone of clearance of pre-selected bacterial isolates A2 and AnNf. Bacterial isolates BTD6, K1 and 4-1H formed area with vaguely defined border parts. Fig. 4 and 5 shows exemplary growth and formation of a zone of clearance of pre-selected isolates BTD6 and BTN1 in comparison. Isolate 15-2 showed very fast growth and clearly defined formation of a zone of clearance (Fig.6).

Fig. 7 shows a graphical display (comparison) of the size of the zone of clearance, zone width in cm, achieved by active bacterial isolates on the selective medium. Other isolates, such as isolates B43, 3-1A, A5, II-1, A4, B42, 2-7A, B41, PO1, BS1, 12-1A and A1 showed a very good growth on the selective medium, but did not form a zone of clearance due to chitin hydrolysis. These strains are apparently able to metabolize chitin, but the produced chitinolytic enzymes are probably less motile in an agar nutrient medium, or they are attached to the microbial cells and the hydrolyzed chitin coincides with the growth area (Fig. 8). Figure 8 shows exemplary growth without formation of a zone of clearance of isolate A4.

### Example 3: Measurement of chitinolytic activity of fungal and bacterial isolates forming a zone of clearance

Bacterial and fungal isolates which were able to hydrolyze the chitin outside of the growth zone, i.e. which formed a zone of clearance, were selected for further studies. In particular, active fungal isolates which showed fast growth and formation of a zone of clearance in the preliminary screening were selected, i.e. fungal isolates Tr1, Tr2, Tr3, Tr4, Tr5, Tr22, H1, H2, H5, H6, H7, CH2, CHM1, CHH, CHO, M1, Chl and Csl.

The ability of the selected fungal isolates to produce chitinolytic enzymes was observed in a liquid nutrient medium having the above-mentioned composition (Table 2), every 24 h for a period of 144 h. The isolates were cultivated in 500 ml jars filled with 200 ml medium placed on a shaking incubator (180 rpm, 28°C). The chitinolytic activity was measured based on the quantity of the reduced groups of chitin, which was determined by the addition of 3,5-dinitrosalicylic acid (DNS-reagent) (Miller G.L., (1959) Use of Dinitrosalicylic Acid Reagent for Determination of Reducing Sugar, Anal. Chem., Vol. 31, No. 3, 426-428). For that purpose, 1 ml cultured liquid supernatant (10 min, 10000 rpm) was added to previously tempered 1 ml suspension of 0.5% colloidal chitin in 0.1 M citrate buffer. During the screening, the suspension had a different pH depending on the cultivated microorganisms, i.e. for the measurement of the enzyme activity of the bacterial culture liquid the pH was set to 6.5 and for the fungal culture liquid the pH was set to 5.5. After the screening, whenever this method was used to determine the chitinolytic activity, only one pH was used, namely a pH of 6.0. The mixture (colloidal chitin-supernatant) was heated for 1 h at 50°C and enzymatic reaction was stopped with the addition of 3 ml DNS-reagent. The test tubes were heated in a boiling water bath for 5 min. The optical density was measured at 575 nm on Spectrophotometer "Spectroquant® Pharo 300" (Merck) after cooling of the test tubes.

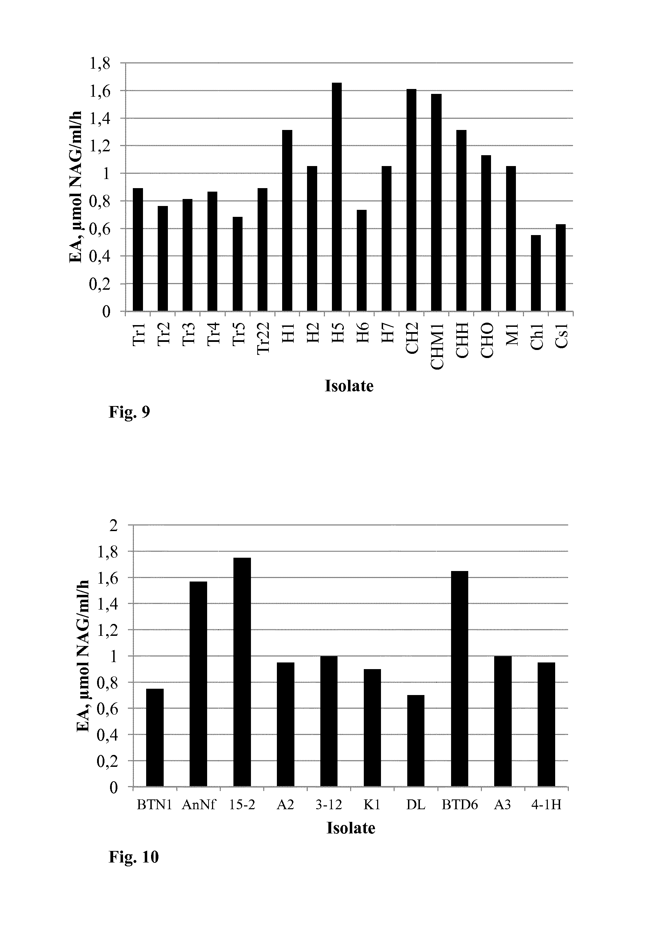

Fig. 9 shows the chitinolytic activity EA of the selected fungal isolates on day 4 of the incubation in µmol *N*-acetyl-D-glucosamine produced per ml and h (NAG/ml/h). Isolates H1, H2, H5, H7, CH2, CHM1, CHH, CHO and M1 had a high chitinolytic activity EA of higher than 1 µmol *N*-acetyl-D-glucosamine per ml and h. Amongst them, fungal isolates H5, CH2 and CHM1 showed the highest chitinolytic activities. Fig. 10 shows the resulting enzyme activity on day 4 of the selected bacterial isolates. Highest chitinolytic activity showed bacterial isolates AnNf, 15-2 and BTD6. Best results were obtained from fungal isolates H5, CH2 and CHM1, and bacterial isolates AnNf, 15-2 and BTD6. These strains were chosen for further analyses.

### Example 4: Dynamics of enzyme synthesis of strain H5

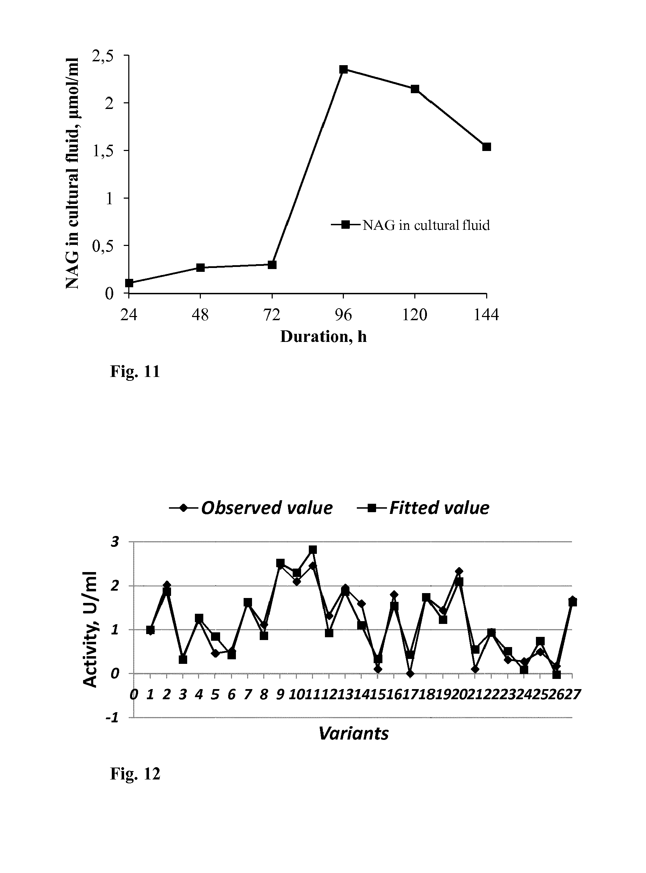

The investigation of the dynamics of enzyme synthesis was started with cultivation of fungal isolate H5, showing the highest chitinolytic activity. The cultivation was performed in a laboratory bioreactor with 11 volume of liquid nutrient medium with the following composition (amounts in g/l): KH₂PO₄ - 0.7, K₂HPO₄ - 0.3, NaCl - 2.0, MgSO₄*7H₂O - 0.5, colloidal chitin - 5.0, high molecular chitosan - 5.0. Quantity of the reducing substances in the culture liquid, i.e. the chitinolytic activity, was determined every 24 h for a period of 144 h as described in Example 3. The data obtained during the performed analyses is shown in Fig. 11. As it can be seen from Fig. 11, the enzyme activity increases between 24 h and 120 h. The level is highest between 72 h and 96 h. The highest activity is achieved around 120 h, after which a low reduction is observed. The reducing substances produced in the nutrient medium are equivalent to the increase in enzyme activity during cultivation. The highest level of accumulation of the reducing substances coincides with the highest level of increase in enzyme activity. After the period of increase, their quantity in the medium decreases very fast.

### Example 5: Molecular taxonomic identification of new isolated microbial strains, in particular of strain Aspergillus flavus H5

Collected samples of wet biomass were generally frozen immediately and then stored at -20°C until further processing for nucleic acid extraction. DNA was extracted from the samples using a bead beating protocol. Generally, the following protocol was used: the sample (0.5 to 1.0 g) was re-suspended in 0.5 ml of sodium dodecyl sulfate-buffer solution (SDS) (200 mM Tris pH 8.0, 20 mM EDTA, 200mMNaCl, 2% SDS) and incubated for 20 min at 70°C. The sample was reciprocated on a Mini-Beadbeater (Biospec) at low speed for 2 min in the presence of 0.3 g of acid-washed zirconium-silica beads (0.1mm in diameter) and phenol. Nucleic acids were precipitated from the supernatant with sodium acetate and isopropanol, then the sample was dried and re-suspended in Tris pH 8.0. Final DNA purification was conducted with a GFX Genomic Blood DNA Purification Kit (Amersham Biosciences, Piscataway, NJ, USA). The quantity and quality of the extracted DNA were determined by measuring the UV absorption spectrum of the DNA solution.

PCR was performed on an Eppendorf Mastercycler using PuReTaq Ready-To-Go™ PCR Beads (Amersham Biosciences, Piscataway, NJ, USA). The PCR reactions were carried out in a final volume of 25 µl, containing 10 pmol of each primer and 50 ng genomic DNA (1 µl). PCR conditions for 16S rDNAs amplification included 95°C for 5 min, followed by 35 cycles: 95°C for 30 s, 55°C for 30 s, 60°C for 30 s and final extension at 72°C for 10 min. PCR products were stored at 4°C. PCR conditions for 18S rDNAs amplification included 95°C for 5 min, followed by 35 cycles: 95°C for 60 s, 65°C for 60 s, 60°C for 30 s and final extension at 72°C for 7 min. PCR products were stored at 4°C. 16S rDNAs were amplified with universal reverse oligonucleotide primer 1492R (5'-GGTTACCTTGTTACGACTT-3') (SEQ ID No:2) and with the forward primer 27F (specific for Bacteria) (5'-AGAGTTTGATCCTGGCTCAG-3') (SEQ ID No:3). 18S rDNAs were amplified with universal primers Pffor (5'-AGGGATGTATTTATTAGATAAAAAATCAA-3') (SEQ ID No:4) and Pfrev (5'-CGCAGTAGTTAGTCTTCAGTAAATC-3') (SEQ ID No:5).

The obtained PCR products were purified by GFX™ PCR DNA and Gel Band Purification Kit (Amersham Biosciences, Piscataway, NJ, USA).

The obtained DNAs (rDNAs) were amplified by PCR from 1 to 50 ng of DNA in reaction mixtures containing (as final concentrations) 1 x PCR buffer II (Perkin-Elmer), 2.5 mM MgCl₂, 200 µM of each deoxynucleoside triphosphate, 300 nM of each forward and reverse primer, and 0.025 U of AmpliTaq Gold DNA polymerase (Perkin-Elmer) per ml. Reaction mixtures were incubated in a Mastercycler Gradient Thermal Cycler (Eppendorf) at 94°C for 12 min (for initial denaturation and activation of AmpliTaq Gold), followed by 25 to 35 cycles at 94°C for 30 s, 50°C for 45 s, and 72°C for 1.5 min, followed by a final extension at 72°C for 12 min.

The obtained PCR products were purified with a GFX™ PCR DNA and Gel Band Purification Kit (Amersham Biosciences, Piscataway, NJ, USA). The sequencing of amplified fragments was performed on an ABI Prism 310 Genetic Analyzer by using BigDye® Terminator Kit version 3.1. The raw data from the Genetic Analyzer was edited by Sequence scanner version 1.0 software (Applied Biosystems, Foster City, CA, USA). Sequences were compared to those in available databases using BLAST (Basic Local Alignment Search Tool) to determine their approximate phylogenetic affiliations. The obtained sequences were paired accordingly and were further analyzed to establish their taxonomic affiliation. The result for strain H5 was as follows:
681 bp 18S rDNA-fragment (SEQ ID No:1):

ATCAATGCCCTTCGGGGCTCCTTGGTGATTCATAATAACTTAACGAATCG
CATGGCCTTGCGCCGGCGATGGTTCATTCAAATTTCTGCCCTATCAACTTT
CGATGGTAGGATAGTGGCCTACCATGGTGGCAACGGGTAACGGGGAATT
AGGGTTCGATTCCGGAGAGGGAGCCTGAGAAACGGCTACCACATCCAAG
GAAGGCAGCAGGCGCGCAAATTACCCAATCCCGACACGGGGAGGTAGTG
ACAATAAATACTGATACGGGGCTCTTTTGGGTCTCGTAATTGGAATGAGT
ACAATCTAAATCCCTTAACGAGGAACAATTGGAGGGCAAGTCTGGTGCC
AGCAGCCGCGGTAATTCCAGCTCCAATAGCGTATATTAAAGTTGTTGCAG
TTAAAAAGCTCGTAGTTGAACCTTGGGTCTGGCTGGCCGGTCCGCCTCAC
CGCGAGTACTGGTCCGGCTGGACCTTTCCTTCTGGGGAACCTCATGGCCT
TCACTGGCTGTGGGGGGAACCAGGACTTTTACTGTGAAAAAATTAGAGT
GTTCAAAGCAGGCCTTTGCTCGAATACATTAGCATGGAATAATAGAATAG

GACGTGCGGTTCTATTTGTTGGTTTCTAGGACCGCCGTAATGATTAATA
GGGATAGTCGGGGGCGTCAGTATTCAGCTGTCAGA

This strain appeared to be closely related to *Aspergillus flavus* strains, since the observed sequence is 100% identical to sequences of the *Aspergillus* strains listed in Table 3 below. The comparison of the data with NCBI Gene Bank Data Base reference sequences was conducted using BLAST. The phylogenetic analysis of rDNA sequences were performed by using ClustalW version 1.7.

The data are for standard nucleotide BLAST analyses are given at the site of the National Center for Biotechnology Information (NCBI) (http://blast.ncbi.nlm.nih.govBlast.cgi#33334374)

**Table 3: Results of BLAST analysis of Aspergillus strains**

| Accession | Description | Max score | Total score | Query coverage | E value | Max. ident. |
|---|---|---|---|---|---|---|
| JN938987.1 | *Aspergillus flavus* strain DAOM 225949 18S ribosomal RNA (SSU) gene, partial sequence | 1254 | 1254 | 99% | 0.0 | 100% |
| JF824683.1 | *Aspergillus flavus* isolate P.tri.Iso1 18S ribosomal RNA gene, partial sequence | 1254 | 1254 | 99% | 0.0 | 100% |
| HQ393872.1 | *Aspergillus flavus* strain PSFW1RH-4 18S ribosomal RNA gene, partial sequence | 1254 | 1254 | 99% | 0.0 | 100% |
| GU953210.1 | *Aspergillus flavus* strain TZ1985 18S ribosomal RNA gene, partial sequence | 1254 | 1254 | 99% | 0.0 | 100% |
| AF548060.1 | *Aspergillus flavus* strain UPSC 1768 18S ribosomal RNA gene, partial sequence | 1254 | 1254 | 99% | 0.0 | 100% |
| D63696.1 | *Aspergillus flavus* DNA for 18S rRNA, partial sequence | 1254 | 1254 | 99% | 0.0 | 100% |

### References:

*Aspergillus flavus* strain TZ1985 18S ribosomal RNA gene, partial sequence.
- 24-APR-2010
NCBI ACCESSION N - GU953210
Tao,B., Zhan,H.X., Ren,H.L. and Jiang,L.X., Biodegradation of fomesafen by strain *Aspergillus flavus* TZ1985isolated from soil (unpublished).
*Aspergillus flavus* strain UPSC 1768 18S ribosomal RNA gene, partial sequence.
- 04-SEP-2003
NCBI ACCESSION N - AF548060
Wu,Z., Tsumura,Y., Blomquist,G. and Wang,X.R., (2003) 18S rRNA gene variation among common airborne fungi, and development of specific oligonucleotide probes for the detection of fungal isolates. Appl. Environ. Microbiol. 69 (9), 5389-5397.
*Aspergillus flavus* DNA for 18S rRNA, partial sequence.
- 13-FEB-1999
NCBI ACCESSION N - D63696
Nikkuni,S., Kosaka,N., Suzuki,C. and Mori,K., (1996) Comparative sequence analysis on the 18S rRNA gene of Aspergillus oryzae, A. sojae, A. flavus, A. parasiticus, A. niger, A. awamori and A. tamarii. J. Gen. Appl. Microbiol. 42, 181-187.

The above identified strain, defined as *Aspergillus flavus* H5, was chosen for further investigation, i.e. optimization of the fermentation process in order to synthesize higher amounts of chitinase H5, due to its properties of high chitinolytic activity and relatively good stability at high temperatures.

### Example 6: Optimization of the fermentation/nutrient medium for strain Aspergillus flavus H5

The optimization process was analyzed using optimal composite experimental design (Kostov G. et al., (2011) Lactic acid production with Lactobacillus casei ssp. rhamnosus NBIMCC 1013: modeling and optimization of the nutrient medium. Eng. Life. Science, Vol. 11, No. 5, 517-527). The initial nutrient medium was composed of (amounts in g/l): peptone - 3.0, glucose - 1.0, K₂HPO₄ - 0.2, yeast extract - 2.0, MgSO₄*7H₂O - 0.1, colloidal chitin - 5.0, high molecular weight chitosan- 5.0. Table 4 shows variation limits of the variation of the compounds chitosan and chitin.

**Table 4: Compound variation limits**

| Compound | | -1 | 0 | +1 |
|---|---|---|---|---|
| LMW, ml 2% solution/l (low molecular weight chitosan) | A | 0 | 10 | 20 |
| HMW, ml 2% solution/l (high molecular weight chitosan) | B | 0 | 10 | 20 |
| Ch, g colloidal chitin/l (colloidal chitin, Sigma - 7170) | c | 0 | 6 | 12 |

LMW and HMW were provided by Yantai Shang Tai Trading Co. Ltd., 71 Janshe Road, Yantai, China. LMW had a maximal viscosity of 200 cps, and HMW had a minimal viscosity of 400 cps.

The design of the experiment was a full factorial experiment that is 3³ without repeats in the center and the points of experiment (dispersion homogeneity was suggested, Table 5).

**Table 5: Matrix of the experiment and estimation of chitinolytic activity results**

| No: | Block | A LMW chitosan | B HMW chitosan | Colloidal chitin | Chitinolytic activity | Calculated value | -95% interval | +95% interval |
|---|---|---|---|---|---|---|---|---|
| 1 | 1 | -1 | -1 | -1 | 0.9692 | 0.998704 | 0.494886 | 1.50252 |
| 2 | 1 | 0 | -1 | -1 | 2.0192 | 1.86827 | 1.42168 | 2.31486 |
| 3 | 1 | 1 | -1 | -1 | 0.3661 | 0.319337 | -0.18448 | 0.823154 |
| 4 | 1 | -1 | 0 | -1 | 1.2217 | 1.26821 | 0.837116 | 1.6993 |
| 5 | 1 | 0 | 0 | -1 | 0.4599 | 0.843826 | 0.542738 | 1.14491 |
| 6 | 1 | 1 | 0 | -1 | 0.5135 | 0.419443 | -0.0116504 | 0.850536 |
| 7 | 1 | -1 | 1 | -1 | 1.6059 | 1.63057 | 1.12675 | 2.13439 |
| 8 | 1 | 0 | 1 | -1 | 1.1167 | 0.865037 | 0.418451 | 1.31162 |
| 9 | 1 | 1 | 1 | -1 | 2.4592 | 2.518 | 2.01419 | 3.02182 |
| 10 | 1 | -1 | -1 | 0 | 2.0951 | 2.29204 | 1.74925 | 2.83484 |
| 11 | 1 | 0 | -1 | 0 | 2.4503 | 2.81786 | 2.37127 | 3.26445 |
| 12 | 1 | 1 | -1 | 0 | 1.3177 | 0.925176 | 0.382381 | 1.46797 |
| 13 | 1 | -1 | 0 | 0 | 1.9566 | 1.86985 | 1.39379 | 2.34591 |
| 14 | 1 | 0 | 0 | 0 | 1.5925 | 1.10171 | 0.800626 | 1.4028 |
| 15 | 1 | 1 | 0 | 0 | 0.1 | 0.333581 | -0.142481 | 0.809644 |
| 16 | 1 | -1 | 1 | 0 | 1.8047 | 1.54051 | 0.997714 | 2.0833 |
| 17 | 1 | 0 | 1 | 0 | 0.0 | 0.431226 | -0.0153603 | 0.877812 |
| 18 | 1 | 1 | 1 | 0 | 1.7355 | 1.74044 | 1.19765 | 2.28324 |
| 19 | 1 | -1 | -1 | 1 | 1.4451 | 1.22617 | 0.722353 | 1.72999 |
| 20 | 1 | 0 | -1 | 1 | 2.3274 | 2.09574 | 1.64915 | 2.54232 |
| 21 | 1 | 1 | -1 | 1 | 0.1 | 0.546804 | 0.0429865 | 1.05062 |
| 22 | 1 | -1 | 0 | 1 | 0.9432 | 0.936876 | 0.505783 | 1.36797 |
| 23 | 1 | 0 | 0 | 1 | 0.3125 | 0.512493 | 0.211404 | 0.813581 |
| 24 | 1 | 1 | 0 | 1 | 0.2742 | 0.0881093 | -0.342984 | 0.519202 |
| 25 | 1 | -1 | 1 | 1 | 0.4934 | 0.740437 | 0.23662 | 1.24425 |
| 26 | 1 | 0 | 1 | 1 | 0.1695 | 0.0250963 | -0.471683 | 0.42149 |
| 27 | 1 | 1 | 1 | 1 | 1.6841 | 1.62787 | 1.12405 | 2.13169 |

A, B, C: coded factors (concentration of LMW chitosan, HMW chitosan, colloidal chitin)
Block: fictive factor for estimation of the free coefficient in the model.

### Analysis Experiment - Var 1 (chitinolytic activity):

Table 6 shows the interaction between the compounds' effects and the chitinolytic activity. Each of the estimated effect and interaction is shown. Also shown is the standard error of each of the effects, which measures their sampling error. The largest variance inflation factor (V.I.F.) equals 5.0. For a perfectly orthogonal design, all of the factors would equal 1. Factors of 10 or larger are usually interpreted as indicating serious confounding amongst the effects. Standard errors are based on total error with 15 d.f. (degrees of freedom, equal to the number of coefficients minus one).

**Table 6: Estimation of the effects in the model**

| Effect | Estimate | Standard Error | V.I.F. |
|---|---|---|---|
| average | 1.10171 | 0.14126 | |
| A:Factor A+AAA | -1.53627 | 0.346014 | 5.0 |
| B:Factor B+BBB | -2.38663 | 0.346014 | 5.0 |
| AB | 0.7834 | 0.18952 | 1.0 |
| BB | 1.04566 | 0.268021 | 1.0 |
| BC | -0.5588 | 0.18952 | 1.0 |
| CC | 0.847111 | 0.268021 | 1.0 |
| AAB | 2.4185 | 0.328258 | 3.0 |
| ABB | 0.9528 | 0.328258 | 3.0 |
| ACC | 0.6875 | 0.328258 | 3.0 |
| BCC | 0.8246 | 0.328258 | 3.0 |
| ccc | -0.331333 | 0.154742 | 1.0 |

### Analysis of Variance for Var 1 (chitinolytic activity):

The ANOVA table (Table 7) partitions the variability in Var_1 into separate pieces for each of the effects. It then tests the statistical significance of each effect is determined by comparing the mean square against an estimate of the experimental error. In this case, 11 effects have P-values less than 0.15, indicating that they are significantly different from zero at the 95.0% confidence level. The R-Squared statistic indicates that the model as fitted explains 90.2769% of the variability in Var_1. The adjusted R-squared statistic, which is more suitable for comparing models with different numbers of independent variables, is 83.1467%. The standard error of the estimate shows the standard deviation of the residuals to be 0.328258. The mean absolute error (MAE) of 0.197889 is the average value of the residuals. The Durbin-Watson (DW) statistic tests determines if there is any significant correlation based on the order in which the residuals occur in the data file. Since the P-value is greater than 5.0%, there is no indication of serial autocorrelation in the residuals at the 5.0% significance level.

**Table 7: Analysis of the variances (ANOVA)**

| Source | Sum of Squares | d.f. | Mean Square | F-Ratio | P-Value |
|---|---|---|---|---|---|
| A:Factor A+AAA | 2.1241 | 1 | 2.1241 | 19.71 | 0.0005 |
| B:Factor B+BBB | 5.12642 | 1 | 5.12642 | 47.58 | 0.0000 |
| AB | 1.84115 | 1 | 1.84115 | 17.09 | 0.0009 |
| BB | 1.64009 | 1 | 1.64009 | 15.22 | 0.0014 |
| BC | 0.936772 | 1 | 0.936772 | 8.69 | 0.0100 |
| cc | 1.0764 | 1 | 1.0764 | 9.99 | 0.0065 |
| AAB | 5.84914 | 1 | 5.84914 | 54.28 | 0.0000 |
| ABB | 0.907828 | 1 | 0.907828 | 8.43 | 0.0109 |
| ACC | 0.472656 | 1 | 0.472656 | 4.39 | 0.0536 |
| BCC | 0.679965 | 1 | 0.679965 | 6.31 | 0.0239 |
| ccc | 0.494018 | 1 | 0.494018 | 4.58 | 0.0491 |
| Total error | 1.6163 | 15 | 0.107753 | | |
| Total (corr.) | 16.6233 | 26 | | | |

| | | | | | |
|---|---|---|---|---|---|
| With R² = 90.2769 %; R² (adjusted for d.f.) = 83.1467 %; Standard Error of Est. = 0.328258; Mean absolute error = 0.197889; Durbin-Watson statistic = 2.60538 (P=0.5962); Lag 1 residual autocorrelation = -0.303938. | | | | | |

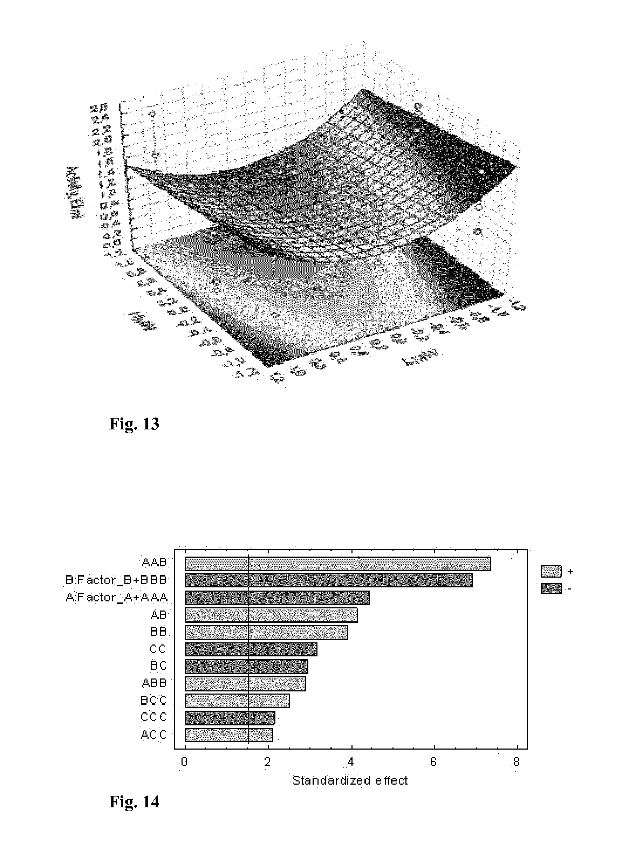

Figure 12 shows the correlation between observed value and fitted value of the chitinolytic activity obtained during optimization of the liquid nutrient medium. Figure 13 shows the Estimated Response Surface of chitinolytic activity calculated during optimization of the liquid nutrient medium, showing an optimum value at 2.786, meaning that the highest activity is observed at this value of the Estimated Response Surface. Figure 14 shows the standardized Pareto Chart of the standardized effect of the concentrations of low molecular weight chitosan, high molecular weight chitosan and colloidal chitin calculated during optimization of the liquid nutrient medium, demonstrating the influence of different factors. This chart helps to eliminate the insignificant factors in the model (model for the nutrient medium composition and chitinolytic activity). Table 8 shows the resulting optimum amounts of each of the three factors A, B, C (LMW, HMW, Ch) in the medium.

**Table 8: Optimal values of the investigated factors**

| Factor | Low | High | Optimum | Optimum g/l |
|---|---|---|---|---|
| Factor_A | -1.0 | 1.0 | 0.00854346 | 10.08 |
| Factor_B | -1.0 | 1.0 | -0.989172 | 0.12 |
| Factor_C | -1.0 | 1.0 | -0.00604116 | 5.96 |

After the optimization procedure, the following optimal concentrations of components of the liquid nutrient medium were obtained:

**Table 9: Optimal concentrations of the nutrient compounds**

| Compound | Amount |
|---|---|
| Peptone | 3.0 g/l |
| Glucose | 1.0 g/l |
| K₂HPO₄ | 0.2 g/l |
| Yeast extract | 2.0 g/l |
| MgSO₄*7H₂O | 0.1 g/l |
| Colloidal chitin | 5.96 g/l |
| Low molecular weight chitosan | 10.08 g/l |
| High molecular weight chitosan | 0.12 g/l |

| | |
|---|---|
| By cultivating *Aspergillus flavus* H5 in above-mentioned medium and placed at 28°C with continuous stirring and aeration after 96 h, an enzyme activity of the culture medium of 6845.24 AU/cm³ is obtained. | |

### Example 7: Partial purification of the enzyme chitinase H5 after growth of the strain Aspergillus flavus H5, producer of chitinase H5

### Example 7a: Ultrafiltration and fractionation (enzyme precipitation)

Initial enzyme purification and concentration was accomplished by means of ultrafiltration (ultrafiltration system DDSRO-Division, Denmark; Module Mini-Lab 10, UF membrane FS81PP, 6000 Da, variation between inlet pressure and outlet pressure was about 1 bar) and vacuum concentration devices known to those skilled in the art. After these procedures, a 5 fold increased enzyme activity was achieved. The vacuum concentrate was then fractionated. In one example, in a first step ethanol was added to achieve a final ethanol concentration of 30% (v/v) in the mixture for ballast protein separation. A second step was the addition of more ethanol to achieve a final ethanol concentration of 80% (v/v) in the mixture for enzyme precipitation. Both steps were performed at 2°C for 30 min at continuous stirring, followed by centrifugation at 4°C, 6000 rpm, for 40 min. In other examples, the fractionation was performed by adding (NH₄)₂SO₄, acetone or isopropanol (compare Fig. 15 to 18, which show the results of the subsequent SEC with the resulting precipitates). The precipitates were water-solubilized and then purified additionally by means of size-exclusion chromatography and ion-exchange chromatography.

### Example 7b: Size-exclusion chromatography (SEC)

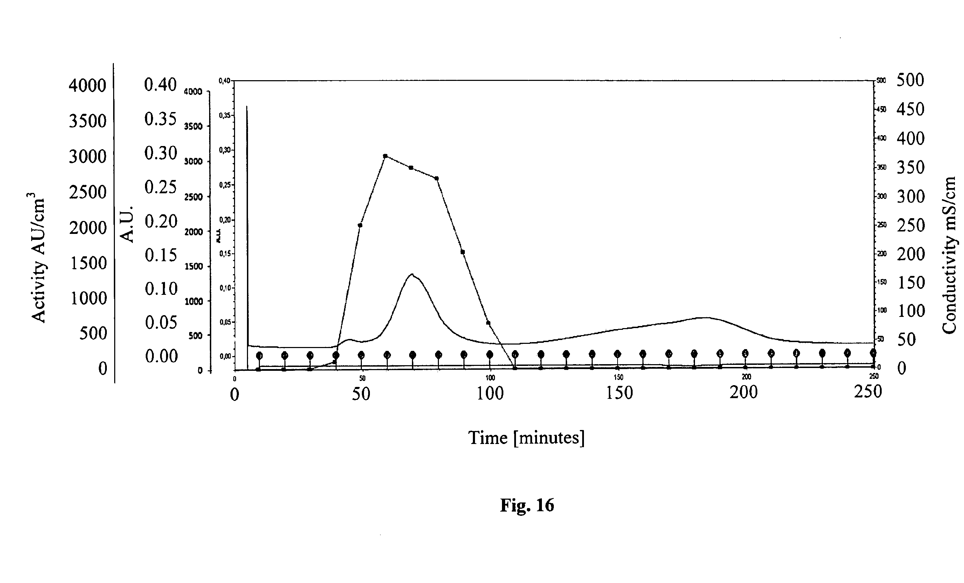

After fractionation, sample separation by means of SEC followed. SEC was performed by using a polyacrylamide gel matrix with a particle size of 90-180 µm ("Bio-Gel^{®} P-100 Gel", Bio-Rad) as stationary phase and 0.05 M NaCl as the mobile phase. Figures 15-18 display SECs after fractionation of chitinase H5. This enzyme has very similar curves depicting the separation process after fractionation of chitinase H5 by saturation with alcohols and acetone (Fig. 16-18). Enzyme activity was determined using the CM-Chitin-RBV method of Example 8c at an incubation temperature of 40°C. The peak possessing an enzyme activity was well separated from other available substances, although the purification level was extremely low. The peak possessing a chitinolytic activity obtained after SEC of a sample previously fractionated with (NH₄)₂SO₄ (Fig. 15) was not well-defined and clearly separated, although its purification level is very similar to those obtained after SECs of alcohols' fractionates (Table 10).

**Table 10: Data of purified chitinase H5 after SEC**

| Solvent/Salt | Fraction Volume, cm³ | Activity, AU/cm³ | Total activity, AU | Protein, µg/cm³ | Specific activity, AU/µg |
|---|---|---|---|---|---|
| (NH₄)₂SO₄ | 0.50 | 2 386.00 | 1 193.00 | 550.00 | 4.34 |
| Acetone | 0.50 | 2 432.00 | 1 216.00 | 244.00 | 9.97 |
| Isopropanol | 0.50 | 2 258.00 | 1 129.00 | 562.00 | 4.02 |
| Ethanol | 0.50 | 2 558.00 | 1 279.00 | 499.00 | 5.13 |

### Example 7c: Ion-exchange chromatography (IEC)

The fractions, collected by SEC, were processed by IEC. IEC was performed with the following conditions: Bio-Scale™ Mini Macro-Prep^{®} High Q Cartridges, Bio-Rad, anion exchange -N⁺(CH₃)₃, ≥ 37 mg BSA as stationary phase, flow rate - 0.5 cm³/min, temperature - 25°C, gradient elution with buffer A and buffer B [buffer A (mobile phase): 25 mM Tris-HCl, pH 8.1; buffer B (elution phase): buffer A + 0.5 M NₐCl]; buffer A, 5 min; buffer A:buffer B=3:1, 5 min; buffer A:buffer B= 1:1, 5 min; buffer A:buffer B=1:3, 5 min. Chromatograms showed that solvents (alcohols and acetone) precipitate the culture liquid in a very similar way (Fig. 19-21). Enzyme activity was determined using the CM-Chitin-RBV method of Example 8c at an incubation temperature of 40°C. The enzyme activity was determined in the first peaks that passed through the ion-exchange column, which means that the enzyme molecule is either positive charged or is a neutral one. Two merged peaks passed through the columns first (Fig. 19-21). Concerning the data regarding the enzyme activity, it can be concluded that chitinase H5 forms the second peak. The data in Table 11 gives information about the specific activity of the enzyme.

**Table 11: Data of partially purified chitinase H5 after IEC**

| Solvent/Salt | Volume, cm³ | Activity, AU/cm³ | Total activity, AU | Protein, µg/cm³ | Specific activity, AU/µg |
|---|---|---|---|---|---|
| (NH₄)₂SO₄ | - | - | - | - | - |
| Acetone | 0.50 | 2 800.00 | 1 400.00 | 840.00 | 3.33 |
| Isopropanol | 0.50 | 3 920.00 | 1 960.00 | 280.00 | 14.00 |
| Ethanol | 0.50 | 3 360.00 | 1 680.00 | 180.00 | 18.67 |

### Example 8: Determination of the enzyme characteristics

### Example 8a: Kinetic constants determination of partially purified chitinase H5

Kₘ and Vₘₐₓ were determined as follows: In order to determine Kₘ and Vₘₐₓ different concentrations of carboxymethyl-chitin-remazol-brilliant-violet (CM-Chitin-RBV, LOEWE Biochemica GmbH, Germany) were used: 0.04, 0.08, 0.12, 0.16 and 0.2 mg/cm³. During this observation standard conditions were applied: chitinase H5 concentration 0.18 mg/cm³, 40°C for 10 min at pH 8.0. Measurement details see Example 8c. The results obtained after this investigation are: Kₘ=752 µg/cm³; Vₘₐₓ=20000 AU/min which indicate a good affinity between chitinase H5 and the substrate. Fig. 23 shows a Lineweaver-Burk plot for determining Kₘ and Vₘₐₓ of chitinase H5.

### Example 8b: Determination of optimum pH value, pH profile of chitinase H5

The pH range of pH from 2 to 12 was investigated for unpurified chitinase H5 (culture liquid) and partially purified chitinase H5 resulting from Example 7. Fig. 24 and 25 show the enzyme behavior under these conditions. It was found that there is a significant difference between the curves of unpurified and partially purified chitinase H5. The unpurified enzyme had one optimum pH value (pH 6.0) (Fig. 24), whereas the partially purified chitinase H5 had two clearly defined optimum pH values: one at pH 3.0 and one at pH 8.0 (Fig. 25). Without being bound by theory, side proteins contained in the culture liquid may influence the enzyme activity of the unpurified chitinase H5. It might be that the purified enzyme has two iso forms which have activity at two different pH values (pH 3.0 and pH 8.0). Moreover, chitinase H5 keeps its activity in a very wide pH range from 3.0 to 8.0 and its activity varies at very low level.

### Example 8c: Determination of optimum temperature of the enzyme action, temperature profile of chitinase H5

The chitinolytic activity of the chitinase H5 was studied in a temperature range from 30°C to 80°C. 200 µl CM-Chitin-RBV (2 mg/ml) and 400 µl 50mM Tris-HCl buffer, pH 8.0, were mixed for 5 minutes. Then 200 µl of enzyme (10 µg/cm³) were added and incubated at 30°C to 80°C for 10 minutes. The reaction was stopped by adding 200 µl 2M HCl. The sample was cooled in a fridge camera for 10 minutes, and then centrifuged for 5 minutes at 10000 g. The supernatant was measured spectrophotometrically at 550 nm against a standard sample prepared by adding 200 µl distillated water instead of enzyme. Chitinase H5 had a maximal enzyme activity at 50°C (Fig. 26 and 27). Crude and partially purified chitinase H5 had the same optimum temperature of enzyme action.

Based on the results of Example 8c, the standard conditions for the CM-Chitin-RBV method were set as follows: chitinase H5 concentration 0.18 mg/cm³, 40°C for 10 min at pH 8.0.

While the invention has been described and illustrated with reference to certain particular embodiments thereof, those skilled in the art will appreciate that various adaptations, changes, modifications, substitutions, deletions, or additions of procedures and protocols may be made without departing from the spirit and scope of the invention. It is intended that the invention be defined by the scope of the claims which follow and that such claims be interpreted as broadly as is reasonable.

## Claims

1. A microbial strain, wherein the strain is *Aspergillus flavus*H5*,* as deposited in the National Bank for Industrial Microorganisms and Cell Cultures (NBIMCC) - Bulgaria under the access no. NBIMCC 8735.

2. Use of the strain of claim 1, for the production of a chitinase.

3. Chitinase, produced by the strain of claim 1.

4. The chitinase of claim 3, **characterized by** one or more parameters selected from a Kₘ in the range of from 640 to 864 µg/cm³, Vₘₐₓ in the range of from 17000 to 23000 AU/min, a Tₘₐₓ in the range of from 30 to 70°C and a first pH optimum in a pH range of from 1.0 to 5.0, and a second pH optimum in a pH range of from 7.0 to 10.0.

5. A medium for culturing a chitinase-producing strain, preferably the strain of claim 1, wherein the medium comprises the following components:
colloidal chitin, low molecular weight chitosan, and high molecular weight chitosan.

6. The medium of claim 5, wherein the medium comprises colloidal chitin in an amount of from 4.0 to 8.0 g/l, low molecular weight chitosan in an amount of from 8.0 to 12.0 g/l, and high molecular weight chitosan in an amount of from 0.08 to 0.4 g/l.

7. The medium of claim 5 or 6, wherein the medium further comprises one or more of following components: peptone, glucose, K₂HPO₄, yeast extract, MgSO₄*7H₂O.

8. The medium of claim 7, wherein the medium comprises peptone in an amount of from 2.0 to 4.0 g/l, glucose in an amount of from 0.2 to 1.8 g/l, K₂HPO₄ in an amount of from 0.1 to 0.3 g/l, yeast extract in an amount of from 1.0 to 3.0 g/l, and MgSO₄*7H₂O in an amount of from 0.02 to 0.18 g/l.

9. Use of the chitinase of claims 3 or 4, in a temporary or permanent anti-contamination coating system.

10. The use of claim 9, wherein the temporary or permanent anti-contamination coating system is applied to a surface for the detachment of chitin-containing debris, preferably insect debris, from said surface.

11. The use of claim 10, wherein the surface is an airfoil, preferably a laminar airfoil.

12. A method for producing a chitinase, comprising the following steps:
a) culturing a chitinase-producing strain in a liquid nutrient medium;
b) separating the nutrient medium from the strain cells; and
c) isolating the chitinase from the cultivation supernatant.

13. The method of claim 12, wherein the liquid nutrient medium is the medium of any one of claims 5 to 8.

14. The method of claim 12 or 13, wherein the chitinase-producing strain is the strain of claim 1.

15. Use of the chitinase of claims 3 or 4 for the degradation of chitin.


SEQUENCE LISTING

<110>  EADS Deutschland GmbH

<120>  Novel Aspergillus sp. strain NBIMCC 8735 and
        method of producing chitinase utilizing the same

<130>  E 13123/KK

<160>  5

<170>  PatentIn version 3.5

<210>  1
<211>  681
<212>  DNA
<213>  Aspergillus flavus


<220>
<221>  misc_feature
<223>  681 bp 18S rDNA-fragment

<400>  1
atcaatgccc ttcggggctc cttggtgatt cataataact taacgaatcg catggccttg      60

cgccggcgat ggttcattca aatttctgcc ctatcaactt tcgatggtag gatagtggcc     120

taccatggtg gcaacgggta acggggaatt agggttcgat tccggagagg gagcctgaga     180

aacggctacc acatccaagg aaggcagcag gcgcgcaaat tacccaatcc cgacacgggg     240

aggtagtgac aataaatact gatacggggc tcttttgggt ctcgtaattg gaatgagtac     300

aatctaaatc ccttaacgag gaacaattgg agggcaagtc tggtgccagc agccgcggta     360

attccagctc caatagcgta tattaaagtt gttgcagtta aaaagctcgt agttgaacct     420

tgggtctggc tggccggtcc gcctcaccgc gagtactggt ccggctggac ctttccttct     480

ggggaacctc atggccttca ctggctgtgg ggggaaccag gacttttact gtgaaaaaat     540

tagagtgttc aaagcaggcc tttgctcgaa tacattagca tggaataata gaataggacg     600

tgcggttcta ttttgttggt ttctaggacc gccgtaatga ttaataggga tagtcggggg     660

cgtcagtatt cagctgtcag a                                               681


<210>  2
<211>  19
<212>  DNA
<213>  Artificial Sequence

<220>
<223>  universal reverse oligonucleotide primer 1492R

<400>  2
ggttaccttg ttacgactt                                                   19


<210>  3
<211>  20
<212>  DNA
<213>  Artificial Sequence

<220>
<223>  forward primer 27F

<400>  3
agagtttgat cctggctcag                                                    20


<210>  4
<211>  29
<212>  DNA
<213>  Artificial Sequence

<220>
<223>  primer PFfor

<400>  4
agggatgtat ttattagata aaaaatcaa                                          29


<210>  5
<211>  25
<212>  DNA
<213>  Artificial Sequence

<220>
<223>  primer PFrev

<400>  5
cgcagtagtt agtcttcagt aaatc                                              25